(19) Europäisches Patentamt
European Patent Office
Office européen des brevets

(11) EP 3 330 350 A1

# (12) EUROPEAN PATENT APPLICATION

(43) Date of publication:
**06.06.2018 Bulletin 2018/23**

(51) Int Cl.:
***C11D 3/386*** *(2006.01)* ***C11D 3/39*** *(2006.01)*

(21) Application number: **17204737.5**

(22) Date of filing: **30.11.2017**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **02.12.2016 EP 16202071**

(71) Applicant: **The Procter & Gamble Company**
**Cincinnati, OH 45202 (US)**

(72) Inventor: **LANT, Neil Joseph**
**Newcastle upon Tyne, NE12 9TS (GB)**

(74) Representative: **Peet, Jillian Wendy**
**Procter & Gamble Technical Centres Limited**
**Whitley Road**
**Longbenton**
**Newcastle upon Tyne**
**NE12 9TS (GB)**

## (54) CLEANING COMPOSITIONS INCLUDING ENDO-BETA-1,6-GALACTANASE ENZYMES AND BLEACH

(57) Cleaning compositions that include a galactanase enzyme, a peroxygen source; and an bleach catalyst and methods of making and using such cleaning compositions to provide stain and soil removal and/or malodour reduction.

EP 3 330 350 A1

SEQUENCE LISTING

<110>  P&G

<120>  CLEANING COMPOSITIONS INCLUDING ENZYME AND BLEACH

<130>  CM04647FM

<160>  14

<170>  PatentIn version 3.5

<210>  1
<211>  463
<212>  PRT
<213>  Streptomyces davawensis

<400>  1

```
Asp Ala Thr Ile Val Ile Asn Pro Gly Thr Arg Tyr Gly Thr Trp Glu
1               5                   10                  15

Gly Trp Gly Thr Ser Leu Ala Trp Trp Gly Asn Val Phe Gly Thr Arg
            20                  25                  30

Asp Asp Phe Ala Asp Leu Phe Phe Thr Thr Lys Ser Val Thr Tyr Asn
        35                  40                  45

Gly Thr Ser Leu Pro Gly Leu Gly Leu Asn Ile Ala Arg Tyr Asn Leu
    50                  55                  60

Gly Ala Cys Ser Trp Asn Ala Val Asn Gly Glu Thr Met Val Lys Ser
65                  70                  75                  80

Pro Asn Ile Pro Ala Phe Lys Gln Ile Glu Gly Phe Trp Gln Asp Trp
                85                  90                  95

Asn Asn Glu Asp Pro Thr Ser Ser Ala Trp Asp Trp Thr Ala Asp Ala
            100                 105                 110

Thr Gln Arg Ala Met Leu Val Lys Ala Thr Gln Arg Gly Ala Val Thr
        115                 120                 125

Glu Leu Phe Ala Asn Ser Pro Met Trp Trp Met Cys Tyr Asn His Asn
    130                 135                 140

Pro Ser Gly Ala Ala Asp Gly Gly Asn Asn Leu Gln Thr Trp Asn Tyr
145                 150                 155                 160

Arg Gln His Ala Ser His Leu Ala Ala Val Ala Leu Tyr Ala Arg Thr
                165                 170                 175
```

Asn Trp Gly Val Asn Phe Ala Thr Val Asp Pro Phe Asn Glu Pro Ala
180 185 190

Ser Ser Trp Trp Thr Ala Ser Gly Thr Gln Glu Gly Cys His Leu Asp
195 200 205

Pro Ala Val Gln Ala Ala Val Leu Pro Tyr Met Arg Ser Glu Leu Asp
210 215 220

Lys Arg Gly Leu Thr Gly Val Arg Ile Ser Ala Ser Asp Glu Thr Asn
225 230 235 240

Tyr Asp Thr Ala Arg Ser Thr Trp Ser Ser Phe Gly Ser Ala Thr Lys
245 250 255

Ala Leu Val Ser Gln Val Asn Val His Gly Tyr Gln Gly Thr Gly Gly
260 265 270

Arg Arg Asp Leu Leu Tyr Thr Asp Val Val Thr Thr Ser Gly Lys Lys
275 280 285

Leu Trp Asn Ser Glu Thr Gly Asp Ser Asp Gly Thr Gly Leu Ser Met
290 295 300

Ala Arg Asn Leu Cys Tyr Asp Phe Arg Trp Leu His Pro Thr Ala Trp
305 310 315 320

Cys Tyr Trp Gln Val Met Asp Pro Ser Thr Gly Trp Ala Met Ile Ala
325 330 335

Tyr Asp Ala Asn Thr Leu Gln Pro Thr Thr Val Gln Pro Lys Tyr Tyr
340 345 350

Val Met Ala Gln Phe Ser Arg His Ile Arg Pro Gly Met Thr Ile Leu
355 360 365

Asp Thr Gly Val Ser Phe Ala Ala Ala Ala Tyr Asp Ala Ser Ala Arg
370 375 380

Arg Leu Val Leu Val Ala Val Asn Thr Ser Thr Ser Pro Gln Thr Phe
385 390 395 400

Thr Phe Asp Leu Ser Arg Phe Thr Thr Val Thr Gly Gly Ser Gly Gly
405 410 415

Leu Val Pro Arg Trp Asn Thr Val Thr Gly Gly Gly Asp Met Tyr Arg
420 425 430

Ala Tyr Thr Asn Thr Tyr Val Thr Gly Lys Ser Val Ser Ala Thr Phe
435 440 445

Ala Ala Gly Ser Val Gln Thr Leu Gln Val Asp Gly Val Thr Thr
450 455 460

<210> 2
<211> 464
<212> PRT
<213> Streptomyces avermitilis

<400> 2

Asp Ala Thr Ile Ala Val Asn Pro Ser Thr Thr Tyr Gly Lys Trp Glu
1 5 10 15

Gly Trp Gly Thr Ser Leu Ala Trp Trp Ala Asn Val Phe Gly Ala Arg
20 25 30

Asp Asp Phe Ala Asp Leu Phe Phe Thr Thr Lys Ser Val Thr Tyr Asn
35 40 45

Gly Arg Thr Leu Pro Gly Leu Gly Leu Asn Ile Ala Arg Tyr Asn Leu
50 55 60

Gly Ala Cys Ser Trp Asn Ser Val Ser Gly Glu Ser Met Val Ala Ser
65 70 75 80

Ala Asn Ile Pro Ala Phe Lys Gln Ile Glu Gly Tyr Trp Gln Asp Trp
85 90 95

Asn Asn Glu Asp Pro Thr Ser Ser Ala Trp Lys Trp Thr Ala Asp Ala
100 105 110

Ala Gln Arg Thr Met Leu Val Lys Ala Thr Ala Arg Gly Ala Thr Thr
115 120 125

Glu Leu Phe Ala Asn Ser Pro Met Trp Trp Met Cys Leu Asn His Asn
130 135 140

Pro Ser Gly Ala Ser Gly Gly Gly Asn Asn Leu Gln Ser Trp Asn Tyr
145 150 155 160

Arg Gln His Ala Ser His Leu Ala Ala Val Ala Leu Tyr Ala Lys Ser
165 170 175

Asn Trp Gly Val Asn Phe Ala Thr Val Asp Pro Phe Asn Glu Pro Ser
180 185 190

```
Ser Ser Trp Trp Thr Ala Thr Gly Thr Gln Glu Gly Cys His Met Asp
        195                 200                 205

Ala Ser Val Gln Ala Ala Val Leu Pro Tyr Leu Arg Ser Glu Leu Asp
    210                 215                 220

Arg Arg Gly Leu Thr Gly Thr Lys Ile Ser Ala Ser Asp Glu Thr Ser
225                 230                 235                 240

Tyr Asp Leu Ala Arg Thr Thr Trp Gly Ser Phe Gly Ser Ser Thr Lys
                245                 250                 255

Ala Leu Val Asn Arg Val Asn Val His Gly Tyr Gln Gly Ser Gly Gly
            260                 265                 270

Arg Arg Asp Leu Leu Tyr Thr Asp Val Val Thr Thr Ala Gly Lys Ala
        275                 280                 285

Leu Trp Asn Ser Glu Thr Gly Asp Ser Asp Gly Thr Gly Leu Thr Leu
    290                 295                 300

Ala Ser Asn Leu Cys Leu Asp Phe Arg Trp Leu His Pro Thr Ala Trp
305                 310                 315                 320

Val Tyr Trp Gln Val Met Asp Pro Ser Ser Gly Trp Ala Met Ile Ala
                325                 330                 335

Tyr Asp Ala Ser Thr Leu Gln Pro Gly Ala Val Gln Thr Lys Tyr Tyr
            340                 345                 350

Val Met Ala Gln Phe Ser Arg His Ile Arg Ala Gly Met Thr Ile Val
        355                 360                 365

Asp Thr Gly Val Gly Tyr Ala Ala Ala Ala Tyr Asp Ala Thr Ala Arg
    370                 375                 380

Arg Leu Val Ile Val Ala Val Asn Thr Ser Thr Ser Ala Gln Thr Leu
385                 390                 395                 400

Thr Phe Asp Leu Ser Arg Phe Ser Thr Val Thr Gly Gly Thr Gly Gly
                405                 410                 415

Leu Val Arg Arg Trp Asn Thr Val Thr Gly Gly Gly Gly Asp Leu Tyr
            420                 425                 430

Ala Ala His Ser Asp Thr Tyr Leu Ser Gly Lys Ser Leu Ser Val Pro
        435                 440                 445
```

```
Phe Ala Ala Gly Ala Val Gln Thr Leu Glu Val Asp Gly Val Thr Val
    450                 455                 460


<210>  3
<211>  458
<212>  PRT
<213>  Trichoderma harzianum

<400>  3

Asp Thr Thr Leu Ser Ile Asp Pro Thr Ser Asn Trp Gly Thr Trp Glu
1               5                   10                  15


Gly Trp Gly Val Ser Leu Ala Trp Trp Ala Lys Ala Phe Gly Asn Arg
            20                  25                  30


Asp Asp Leu Ala Asn Val Phe Phe Thr Arg Asn Asn Gln Val Ile Asn
        35                  40                  45


Gly Gln Asn Leu Pro Gly Leu Gly Phe Asn Ile Ala Arg Tyr Asn Ala
    50                  55                  60


Gly Ala Cys Ser Thr Asn Thr Tyr Asn Gly Ser Ser Met Val Val Ser
65                  70                  75                  80


Ser Ser Ile Lys Pro Ser Arg Gln Val Asp Gly Tyr Trp Leu Asp Trp
                85                  90                  95


Ala Ser Thr Asp Pro Ala Ser Ser Ser Trp Asn Trp Asn Val Asp Ala
            100                 105                 110


Asn Gln Arg Ala Met Leu Gln Lys Ala Lys Ala Asn Gly Ala Asn Ile
        115                 120                 125


Phe Glu Leu Phe Ser Asn Ser Pro Met Trp Trp Met Cys Leu Asn His
    130                 135                 140


Asn Pro Ser Gly Ser Gly Ser Ser Asp Asn Leu Gln Ser Trp Asn Tyr
145                 150                 155                 160


Gln Asn His Ala Val Tyr Leu Ala Asn Ile Ala Gln His Ala Gln Gln
                165                 170                 175


Asn Trp Gly Ile Gln Phe Gln Ser Val Glu Ala Phe Asn Glu Pro Ser
            180                 185                 190


Ser Gly Trp Gly Pro Thr Gly Thr Gln Glu Gly Cys His Phe Ala Val
        195                 200                 205
```

Ser Thr Met Ala Thr Val Ile Gly Tyr Leu Asn Thr Glu Leu Ala Gln
    210                 215                 220

Arg Gly Leu Ser Ser Phe Ile Ser Ala Ser Asp Glu Thr Ser Tyr Asp
225                 230                 235                 240

Leu Ala Ile Ser Thr Trp Gln Gly Leu Gly Ser Ser Ala Gln Asn Ala
                245                 250                 255

Val Lys Arg Val Asn Val His Gly Tyr Gln Gly Gly Gly Gly Arg Arg
            260                 265                 270

Asp Thr Leu Tyr Ser Leu Val Ser Gln Ala Gly Lys Arg Leu Trp Asn
        275                 280                 285

Ser Glu Tyr Gly Asp Ala Asp Ala Ser Gly Lys Ser Met Tyr Thr Asn
    290                 295                 300

Leu Leu Leu Asp Phe Thr Trp Leu His Pro Thr Ala Trp Val Tyr Trp
305                 310                 315                 320

Gln Ala Ile Asp Gly Ser Gly Trp Gly Leu Ile Val Gly Asp Asn Asp
                325                 330                 335

Gln Leu Thr Leu Ser Ser Ala Ser Thr Lys Tyr Phe Val Leu Ala Gln
            340                 345                 350

Leu Thr Arg His Ile Arg Pro Gly Met Gln Ile Leu Thr Thr Pro Asp
        355                 360                 365

Gly Asn Thr Val Ala Ala Tyr Asp Ser Gly Ser Gln Lys Leu Val Ile
    370                 375                 380

Val Ala Ala Asn Trp Gly Ser Ala Gln Thr Ile Thr Phe Asp Leu Thr
385                 390                 395                 400

Arg Ala Lys Thr Ala Gly Ser Asn Gly Ala Thr Val Pro Arg Trp Ser
                405                 410                 415

Thr Gln Thr Ser Gly Gly Asp Gln Tyr Lys Ser Tyr Ser Asp Thr Lys
            420                 425                 430

Ile Asn Asn Gly Lys Phe Ser Val Ser Phe Ser Thr Gly Gln Val Gln
        435                 440                 445

Thr Phe Glu Ile Ser Gly Val Val Leu Lys

```
    450                 455


<210>  4
<211>  109
<212>  PRT
<213>  Bacillus licheniformis

<400>  4

Ala Arg Tyr Asp Asp Val Leu Tyr Phe Pro Ala Ser Arg Tyr Pro Glu
1               5                   10                  15


Thr Gly Ala His Ile Ser Asp Ala Ile Lys Ala Gly His Ala Asp Val
            20                  25                  30


Cys Thr Ile Glu Arg Ser Gly Ala Asp Lys Arg Arg Gln Glu Ser Leu
        35                  40                  45


Lys Gly Ile Pro Thr Lys Pro Gly Phe Asp Arg Asp Glu Trp Pro Met
    50                  55                  60


Ala Met Cys Glu Glu Gly Gly Lys Gly Ala Ser Val Arg Tyr Val Ser
65                  70                  75                  80


Ser Ser Asp Asn Arg Gly Ala Gly Ser Trp Val Gly Asn Arg Leu Asn
                85                  90                  95


Gly Tyr Ala Asp Gly Thr Arg Ile Leu Phe Ile Val Gln
            100                 105


<210>  5
<211>  109
<212>  PRT
<213>  Bacillus subtilis

<400>  5

Ala Ser Ser Tyr Asp Lys Val Leu Tyr Phe Pro Leu Ser Arg Tyr Pro
1               5                   10                  15


Glu Thr Gly Ser His Ile Arg Asp Ala Ile Ala Glu Gly His Pro Asp
            20                  25                  30


Ile Cys Thr Ile Asp Asp Gly Ala Asp Lys Arg Arg Glu Glu Ser Leu
        35                  40                  45


Lys Gly Ile Pro Thr Lys Pro Gly Tyr Asp Arg Asp Glu Trp Pro Met
    50                  55                  60


Ala Val Cys Glu Glu Gly Gly Ala Gly Ala Asp Val Arg Tyr Val Thr
65                  70                  75                  80
```

```
Pro Ser Asp Asn Arg Gly Ala Gly Ser Trp Val Gly Asn Gln Met Ser
                85                  90                  95

Ser Tyr Pro Asp Gly Thr Arg Val Leu Phe Ile Val Gln
            100                 105
```

<210> 6
<211> 109
<212> PRT
<213> Bacillus licheniformis

<400> 6

```
Ala Arg Tyr Asp Asp Ile Leu Tyr Phe Pro Ala Ser Arg Tyr Pro Glu
1               5                   10                  15

Thr Gly Ala His Ile Ser Asp Ala Ile Lys Ala Gly His Ser Asp Val
            20                  25                  30

Cys Thr Ile Glu Arg Ser Gly Ala Asp Lys Arg Arg Gln Glu Ser Leu
        35                  40                  45

Lys Gly Ile Pro Thr Lys Pro Gly Phe Asp Arg Asp Glu Trp Pro Met
    50                  55                  60

Ala Met Cys Glu Glu Gly Gly Lys Gly Ala Ser Val Arg Tyr Val Ser
65                  70                  75                  80

Ser Ser Asp Asn Arg Gly Ala Gly Ser Trp Val Gly Asn Arg Leu Ser
                85                  90                  95

Gly Phe Ala Asp Gly Thr Arg Ile Leu Phe Ile Val Gln
            100                 105
```

<210> 7
<211> 204
<212> PRT
<213> Aspergillus oryzae

<400> 7

```
Lys Thr Gly Ser Gly Asp Ser Gln Ser Asp Pro Ile Lys Ala Asp Leu
1               5                   10                  15

Glu Val Lys Gly Gln Ser Ala Leu Pro Phe Asp Val Asp Cys Trp Ala
            20                  25                  30

Ile Leu Cys Lys Gly Ala Pro Asn Val Leu Gln Arg Val Asn Glu Lys
        35                  40                  45
```

```
Thr Lys Asn Ser Asn Arg Asp Arg Ser Gly Ala Asn Lys Gly Pro Phe
    50                  55                  60

Lys Asp Pro Gln Lys Trp Gly Ile Lys Ala Leu Pro Pro Lys Asn Pro
65                  70                  75                  80

Ser Trp Ser Ala Gln Asp Phe Lys Ser Pro Glu Glu Tyr Ala Phe Ala
                85                  90                  95

Ser Ser Leu Gln Gly Gly Thr Asn Ala Ile Leu Ala Pro Val Asn Leu
            100                 105                 110

Ala Ser Gln Asn Ser Gln Gly Gly Val Leu Asn Gly Phe Tyr Ser Ala
        115                 120                 125

Asn Lys Val Ala Gln Phe Asp Pro Ser Lys Pro Gln Gln Thr Lys Gly
    130                 135                 140

Thr Trp Phe Gln Ile Thr Lys Phe Thr Gly Ala Ala Gly Pro Tyr Cys
145                 150                 155                 160

Lys Ala Leu Gly Ser Asn Asp Lys Ser Val Cys Asp Lys Asn Lys Asn
                165                 170                 175

Ile Ala Gly Asp Trp Gly Phe Asp Pro Ala Lys Trp Ala Tyr Gln Tyr
            180                 185                 190

Asp Glu Lys Asn Asn Lys Phe Asn Tyr Val Gly Lys
        195                 200


<210>  8
<211>  188
<212>  PRT
<213>  Trichoderma harzianum

<400>  8

Ala Pro Ala Pro Met Pro Thr Pro Pro Gly Ile Pro Thr Glu Ser Ser
1               5                   10                  15

Ala Arg Thr Gln Leu Ala Gly Leu Thr Val Ala Val Ala Gly Ser Gly
            20                  25                  30

Thr Gly Tyr Ser Arg Asp Leu Phe Pro Thr Trp Asp Ala Ile Ser Gly
        35                  40                  45

Asn Cys Asn Ala Arg Glu Tyr Val Leu Lys Arg Asp Gly Glu Gly Val
    50                  55                  60
```

Gln Val Asn Asn Ala Cys Glu Ser Gln Ser Gly Thr Trp Ile Ser Pro
65 70 75 80

Tyr Asp Asn Ala Ser Phe Thr Asn Ala Ser Ser Leu Asp Ile Asp His
85 90 95

Met Val Pro Leu Lys Asn Ala Trp Ile Ser Gly Ala Ser Ser Trp Thr
100 105 110

Thr Ala Gln Arg Glu Ala Leu Ala Asn Asp Val Ser Arg Pro Gln Leu
115 120 125

Trp Ala Val Ser Ala Ser Ala Asn Arg Ser Lys Gly Asp Arg Ser Pro
130 135 140

Asp Gln Trp Lys Pro Pro Leu Thr Ser Phe Tyr Cys Thr Tyr Ala Lys
145 150 155 160

Ser Trp Ile Asp Val Lys Ser Phe Tyr Lys Leu Thr Ile Thr Ser Ala
165 170 175

Glu Lys Thr Ala Leu Ser Ser Met Leu Asp Thr Cys
180 185

<210> 9
<211> 361
<212> PRT
<213> Aggregatibacter actinomycetemcomitans

<400> 9

Asn Cys Cys Val Lys Gly Asn Ser Ile Tyr Pro Gln Lys Thr Ser Thr
1 5 10 15

Lys Gln Thr Gly Leu Met Leu Asp Ile Ala Arg His Phe Tyr Ser Pro
20 25 30

Glu Val Ile Lys Ser Phe Ile Asp Thr Ile Ser Leu Ser Gly Gly Asn
35 40 45

Phe Leu His Leu His Phe Ser Asp His Glu Asn Tyr Ala Ile Glu Ser
50 55 60

His Leu Leu Asn Gln Arg Ala Glu Asn Ala Val Gln Gly Lys Asp Gly
65 70 75 80

Ile Tyr Ile Asn Pro Tyr Thr Gly Lys Pro Phe Leu Ser Tyr Arg Gln
85 90 95

```
Leu Asp Asp Ile Lys Ala Tyr Ala Lys Ala Lys Gly Ile Glu Leu Ile
            100                 105                 110

Pro Glu Leu Asp Ser Pro Asn His Met Thr Ala Ile Phe Lys Leu Val
        115                 120                 125

Gln Lys Asp Arg Gly Val Lys Tyr Leu Gln Gly Leu Lys Ser Arg Gln
    130                 135                 140

Val Asp Asp Glu Ile Asp Ile Thr Asn Ala Asp Ser Ile Thr Phe Met
145                 150                 155                 160

Gln Ser Leu Met Ser Glu Val Ile Asp Ile Phe Gly Asp Thr Ser Gln
                165                 170                 175

His Phe His Ile Gly Gly Asp Glu Phe Gly Tyr Ser Val Glu Ser Asn
            180                 185                 190

His Glu Phe Ile Thr Tyr Ala Asn Lys Leu Ser Tyr Phe Leu Glu Lys
        195                 200                 205

Lys Gly Leu Lys Thr Arg Met Trp Asn Asp Gly Leu Ile Lys Asn Thr
    210                 215                 220

Phe Glu Gln Ile Asn Pro Asn Ile Glu Ile Thr Tyr Trp Ser Tyr Asp
225                 230                 235                 240

Gly Asp Thr Gln Asp Lys Asn Glu Ala Ala Glu Arg Arg Asp Met Arg
                245                 250                 255

Val Ser Leu Pro Glu Leu Leu Ala Lys Gly Phe Thr Val Leu Asn Tyr
            260                 265                 270

Asn Ser Tyr Tyr Leu Tyr Ile Val Pro Lys Ala Ser Pro Thr Phe Ser
        275                 280                 285

Gln Asp Ala Ala Phe Ala Ala Lys Asp Val Ile Lys Asn Trp Asp Leu
    290                 295                 300

Gly Val Trp Asp Gly Arg Asn Thr Lys Asn Arg Val Gln Asn Thr His
305                 310                 315                 320

Glu Ile Ala Gly Ala Ala Leu Ser Ile Trp Gly Glu Asp Ala Lys Ala
                325                 330                 335

Leu Lys Asp Glu Thr Ile Gln Lys Asn Thr Lys Ser Leu Leu Glu Ala
            340                 345                 350
```

```
Val Ile His Lys Thr Asn Gly Asp Glu
        355                 360


<210>  10
<211>  541
<212>  PRT
<213>  Ascobolus stictoideus

<400>  10

Gln Thr Tyr Thr Leu Glu Ala Glu Ala Gly Thr Leu Thr Gly Val Thr
1               5                   10                  15


Val Met Asn Glu Ile Ala Gly Phe Ser Gly Thr Gly Tyr Val Gly Gly
            20                  25                  30


Trp Asp Glu Asp Ala Asp Thr Val Ser Leu Thr Phe Thr Ser Asp Ala
        35                  40                  45


Thr Lys Leu Tyr Asp Val Lys Ile Arg Tyr Ser Gly Pro Tyr Gly Ser
    50                  55                  60


Lys Tyr Thr Arg Ile Ser Tyr Asn Gly Ala Thr Gly Gly Asp Ile Ser
65                  70                  75                  80


Leu Pro Glu Thr Thr Glu Trp Ala Thr Val Asn Ala Gly Gln Ala Leu
                85                  90                  95


Leu Asn Ala Gly Ser Asn Thr Ile Lys Leu His Asn Asn Trp Gly Trp
            100                 105                 110


Tyr Leu Ile Asp Ala Val Ile Leu Thr Pro Ser Val Pro Arg Pro Pro
        115                 120                 125


His Gln Val Thr Asp Ala Leu Val Asn Thr Asn Ser Asn Ala Val Thr
    130                 135                 140


Lys Gln Leu Met Lys Phe Leu Val Ser Lys Tyr His Lys Ala Tyr Ile
145                 150                 155                 160


Thr Gly Gln Gln Glu Leu His Ala His Gln Trp Val Glu Lys Asn Val
                165                 170                 175


Gly Lys Ser Pro Ala Ile Leu Gly Leu Asp Phe Met Asp Tyr Ser Pro
            180                 185                 190


Ser Arg Val Glu Phe Gly Thr Thr Ser Gln Ala Val Glu Gln Ala Ile
        195                 200                 205
```

```
Asp Phe Asp Lys Arg Gly Gly Ile Val Thr Phe Ala Trp His Trp Asn
    210                 215                 220

Ala Pro Ser Gly Leu Ile Asn Thr Pro Gly Ser Glu Trp Trp Arg Gly
225                 230                 235                 240

Phe Tyr Thr Glu His Thr Thr Phe Asp Val Ala Ala Ala Leu Gln Asn
                245                 250                 255

Thr Thr Asn Ala Asn Tyr Asn Leu Leu Ile Arg Asp Ile Asp Ala Ile
            260                 265                 270

Ala Val Gln Leu Lys Arg Leu Gln Thr Ala Gly Val Pro Val Leu Trp
        275                 280                 285

Arg Pro Leu His Glu Ala Glu Gly Gly Trp Phe Trp Trp Gly Ala Lys
    290                 295                 300

Gly Pro Glu Pro Ala Lys Lys Leu Tyr Lys Ile Leu Tyr Asp Arg Leu
305                 310                 315                 320

Thr Asn Tyr His Lys Leu Asn Asn Leu Ile Trp Val Trp Asn Ser Val
                325                 330                 335

Ala Lys Asp Trp Tyr Pro Gly Asp Glu Ile Val Asp Val Leu Ser Phe
            340                 345                 350

Asp Ser Tyr Pro Ala Gln Pro Gly Asp His Gly Pro Val Ser Ala Gln
        355                 360                 365

Tyr Asn Ala Leu Val Glu Leu Gly Lys Asp Lys Lys Leu Ile Ala Ala
    370                 375                 380

Thr Glu Val Gly Thr Ile Pro Asp Pro Asp Leu Met Gln Leu Tyr Glu
385                 390                 395                 400

Ser Tyr Trp Ser Phe Phe Val Thr Trp Glu Gly Glu Phe Ile Glu Asn
                405                 410                 415

Gly Val His Asn Ser Leu Glu Phe Leu Lys Lys Leu Tyr Asn Asn Ser
            420                 425                 430

Phe Val Leu Asn Leu Asp Thr Ile Gln Gly Trp Lys Asn Gly Ala Gly
        435                 440                 445

Ser Ser Thr Thr Thr Val Lys Ser Thr Thr Thr Thr Pro Thr Thr Thr
```

```
    450                 455                 460

Ile Lys Ser Thr Thr Thr Thr Pro Val Thr Thr Pro Thr Thr Val Lys
465                 470                 475                 480

Thr Thr Thr Thr Pro Thr Thr Thr Ala Thr Thr Val Lys Ser Thr Thr
                485                 490                 495

Thr Thr Ala Gly Pro Thr Pro Thr Ala Val Ala Gly Arg Trp Gln Gln
            500                 505                 510

Cys Gly Gly Ile Gly Phe Thr Gly Pro Thr Thr Cys Glu Ala Gly Thr
        515                 520                 525

Thr Cys Asn Val Leu Asn Pro Tyr Tyr Ser Gln Cys Leu
    530                 535                 540


<210>  11
<211>  526
<212>  PRT
<213>  Chaetomium virescens

<400>  11

Pro Arg Asp Pro Gly Ala Thr Ala Arg Thr Phe Glu Ala Glu Asp Ala
1               5                   10                  15

Thr Leu Ala Gly Thr Asn Val Asp Thr Ala Leu Ser Gly Phe Thr Gly
            20                  25                  30

Thr Gly Tyr Val Thr Gly Phe Asp Gln Ala Ala Asp Lys Val Thr Phe
        35                  40                  45

Thr Val Asp Ser Ala Ser Thr Glu Leu Tyr Asp Leu Ser Ile Arg Val
    50                  55                  60

Ala Ala Ile Tyr Gly Asp Lys Arg Thr Ser Val Val Leu Asn Gly Gly
65                  70                  75                  80

Ala Ser Ser Glu Val Tyr Phe Pro Ala Gly Glu Thr Trp Thr Asn Val
                85                  90                  95

Ala Ala Gly Gln Leu Leu Leu Asn Gln Gly Ser Asn Thr Ile Asp Ile
            100                 105                 110

Val Ser Asn Trp Gly Trp Tyr Leu Ile Asp Ser Ile Thr Leu Thr Pro
        115                 120                 125

Ser Thr Pro Arg Pro Ala His Gln Ile Asn Glu Ala Pro Val Asn Ala
```

130 135 140

Ala Ala Asp Lys Asn Ala Lys Ala Leu Tyr Ser Tyr Leu Arg Ser Ile
145 150 155 160

Tyr Gly Lys Lys Ile Leu Ser Gly Gln Gln Glu Leu Ser Leu Ser Asn
165 170 175

Trp Ile Ala Gln Gln Thr Gly Lys Thr Pro Ala Leu Val Ser Val Asp
180 185 190

Leu Met Asp Tyr Ser Pro Ser Arg Val Glu Arg Gly Thr Val Gly Thr
195 200 205

Ala Val Glu Glu Ala Ile Gln His His Asn Arg Gly Gly Ile Val Ser
210 215 220

Val Leu Trp His Trp Asn Ala Pro Thr Gly Leu Tyr Asp Thr Glu Glu
225 230 235 240

His Arg Trp Trp Ser Gly Phe Tyr Thr Ser Ala Thr Asp Phe Asp Val
245 250 255

Ala Ala Ala Leu Ser Ser Thr Thr Asn Ala Asn Tyr Thr Leu Leu Ile
260 265 270

Arg Asp Ile Asp Ala Ile Ala Val Gln Leu Lys Arg Leu Gln Ser Ala
275 280 285

Gly Val Pro Val Leu Phe Arg Pro Leu His Glu Ala Glu Gly Gly Trp
290 295 300

Phe Trp Trp Gly Ala Lys Gly Pro Glu Pro Ala Lys Lys Leu Trp Gly
305 310 315 320

Ile Leu Tyr Asp Arg Val Thr Asn His His Gln Ile Asn Asn Leu Leu
325 330 335

Trp Val Trp Asn Ser Ile Leu Pro Glu Trp Tyr Pro Gly Asp Ala Thr
340 345 350

Val Asp Ile Leu Ser Ala Asp Val Tyr Ala Gln Gly Asn Gly Pro Met
355 360 365

Ser Thr Gln Tyr Asn Gln Leu Ile Glu Leu Gly Lys Asp Lys Lys Met
370 375 380

Ile Ala Ala Ala Glu Val Gly Ala Ala Pro Leu Pro Asp Leu Leu Gln
385 390 395 400

Ala Tyr Glu Ala His Trp Leu Trp Phe Thr Val Trp Gly Asp Ser Phe
405 410 415

Ile Asn Asn Ala Asp Trp Asn Ser Leu Asp Thr Leu Lys Lys Val Tyr
420 425 430

Thr Ser Asp Tyr Val Leu Thr Leu Asp Glu Ile Gln Gly Trp Gln Gly
435 440 445

Ser Thr Pro Ser Ala Thr Thr Thr Ser Ser Thr Thr Thr Pro Ser Ala
450 455 460

Thr Thr Thr Thr Thr Thr Pro Ser Thr Thr Ala Thr Thr Ala Thr Pro
465 470 475 480

Ser Ala Thr Thr Thr Ala Ser Pro Val Thr Tyr Ala Glu His Trp Gly
485 490 495

Gln Cys Ala Gly Lys Gly Trp Thr Gly Pro Thr Thr Cys Arg Pro Pro
500 505 510

Tyr Thr Cys Lys Tyr Gln Asn Asp Trp Tyr Ser Gln Cys Leu
515 520 525

<210> 12
<211> 452
<212> PRT
<213> Preussia aemulans

<400> 12

Gln Thr Val Ile Tyr Gln Ala Glu Gln Ala Lys Leu Ser Gly Val Thr
1 5 10 15

Val Glu Phe Ser Ile Ile Lys Gln Val Val Gly Thr Gly Tyr Val Glu
20 25 30

Gly Phe Asp Glu Ser Thr Asp Ser Ile Thr Phe Thr Val Glu Ser Thr
35 40 45

Thr Ala Ala Leu Tyr Asp Leu Ala Leu Thr Tyr Asn Gly Pro Tyr Gly
50 55 60

Asp Lys Tyr Thr Asn Val Val Leu Asn Asn Ala Ala Gly Ser Gln Val
65 70 75 80

Ser Leu Pro Ala Thr Thr Ala Trp Thr Thr Val Pro Ala Gly Gln Val
85 90 95

Leu Leu Asn Ala Gly Ala Asn Thr Ile Gln Ile Gln Asn Asn Trp Gly
100 105 110

Trp Tyr Leu Val Asp Ser Ile Ser Leu Lys Pro Ala Ala Thr Arg Gly
115 120 125

Ala His Gln Ile Thr Thr Lys Pro Val Asn Lys Asn Ala Asn Ser Asp
130 135 140

Ala Lys Ala Leu Leu Lys Tyr Leu Gly Ser Ile Tyr Gly Lys Lys Ile
145 150 155 160

Leu Ser Gly Gln Gln Asp Leu Ser Ser Leu Asp Trp Val Thr Lys Asn
165 170 175

Val Gly Lys Thr Pro Ala Val Leu Gly Leu Asp Thr Met Asp Tyr Ser
180 185 190

Glu Ser Arg Lys Ser Arg Gly Ala Val Ser Thr Asp Val Asp Lys Ala
195 200 205

Ile Ala Phe Ala Lys Lys Gly Gly Ile Val Thr Phe Cys Trp His Trp
210 215 220

Gly Ala Pro Thr Gly Leu Phe Asp Ser Ala Ala Gln Pro Trp Tyr Arg
225 230 235 240

Gly Phe Tyr Thr Asp Ala Thr Asp Phe Asn Ile Glu Thr Ala Leu Lys
245 250 255

Asp Thr Thr Asn Ala Asn Tyr Thr Leu Leu Met Lys Asp Ile Asp Thr
260 265 270

Ile Ala Val Gln Leu Lys Lys Leu Gln Asp Ala Gly Val Pro Val Ile
275 280 285

Trp Arg Pro Leu His Glu Ala Glu Gly Gly Trp Phe Trp Trp Gly Ala
290 295 300

Lys Gly Pro Glu Pro Ala Lys Lys Leu Trp Lys Ile Met Tyr Asp Arg
305 310 315 320

Leu Thr Asn Gln His Gly Leu Asn Asn Leu Val Trp Thr Trp Asn Ser
325 330 335

Val Ala Pro Asn Trp Tyr Pro Gly Asp Asp Thr Val Asp Ile Val Ser
340 345 350

Ala Asp Thr Tyr Ser Gln Gly Asp His Gly Pro Ile Ser Ala Thr Tyr
355 360 365

Asn Asn Leu Leu Ala Leu Thr Asn Asp Thr Lys Ile Ile Ala Ala Ala
370 375 380

Glu Ile Gly Ser Val Met Glu Pro Ala Gln Leu Gln Ala Tyr Gln Ala
385 390 395 400

Asp Trp Val Tyr Phe Cys Val Trp Ser Gly Glu Phe Ile Asp Gly Gly
405 410 415

Val Trp Asn Ser Leu Asp Phe Leu Lys Lys Val Tyr Asn Asp Pro Tyr
420 425 430

Val Leu Thr Leu Asp Glu Ile Gln Gly Trp Lys Thr Ala Arg Gly Lys
435 440 445

Pro Arg Val Ser
450

<210> 13
<211> 312
<212> PRT
<213> Yunnania penicillata

<400> 13

Ala Pro Ser Thr Thr Pro Val Asn Glu Lys Ala Thr Asp Ala Ala Lys
1 5 10 15

Asn Leu Leu Ser Tyr Leu Val Glu Gln Ala Ala Asn Gly Val Thr Leu
20 25 30

Ser Gly Gln Gln Asp Leu Glu Ser Ala Gln Trp Val Ser Asp Asn Val
35 40 45

Gly Lys Trp Pro Ala Ile Leu Gly Ile Asp Phe Met Asp Tyr Ser Pro
50 55 60

Ser Arg Val Glu Tyr Gly Ala Val Gly Ser Thr Val Pro Asp Ala Ile
65 70 75 80

Ser Tyr Asp Ser Asp Gly Gly Ile Val Thr Phe Cys Trp His Trp Gly
85 90 95

```
Ser Pro Ser Gly Thr Tyr Asn Thr Thr Asp Gln Pro Trp Trp Ser Asn
            100                 105                 110

Phe Tyr Thr Glu Ala Thr Ala Phe Asp Ile Ala Ala Ala Met Asp Asp
        115                 120                 125

Pro Asp Ser Ala Asp Tyr Asn Leu Leu Val Arg Asp Ile Asp Ala Ile
    130                 135                 140

Ser Glu Leu Leu Leu Gln Leu Gln Asp Leu Asp Ile Pro Ile Leu Trp
145                 150                 155                 160

Arg Pro Leu His Glu Ala Glu Gly Gly Trp Phe Trp Trp Gly Ala Lys
                165                 170                 175

Gly Pro Glu Ala Cys Ile Ala Leu Tyr Arg Leu Met Phe Asp Arg Met
            180                 185                 190

Thr Asn His His Gly Leu Asn Asn Leu Leu Trp Val Trp Asn Ser Val
        195                 200                 205

Asp Pro Ser Trp Tyr Pro Gly Asn Asp Val Val Asp Ile Val Ser Ala
    210                 215                 220

Asp Ile Tyr Ala Asp Ala Gly Asp His Ser Pro Gln Glu Glu Thr Phe
225                 230                 235                 240

Ala Ser Leu Gln Ser Leu Thr Gly Asp Thr Lys Leu Val Ala Leu Gly
                245                 250                 255

Glu Val Gly Asn Ile Pro Asp Pro Ala Ser Thr Gly Gly Val Ala Asp
            260                 265                 270

Trp Ala Tyr Trp Val Thr Trp Asn Gly Asp Phe Ile Lys Gly Glu Asp
        275                 280                 285

Tyr Asn Pro Leu Glu Tyr Lys Lys Glu Val Phe Ser Ala Glu Asn Ile
    290                 295                 300

Ile Thr Arg Asp Glu Val Asp Val
305                 310
```

<210> 14
<211> 327
<212> PRT
<213> Myrothecium roridum

<400> 14

```
Gly Thr Ile Glu Asn Arg Gln Trp Leu Thr Tyr Asn Pro Val Asp Ser
1               5                   10                  15

Ala Ala Thr Thr Glu Ala Arg Ala Leu Leu Arg Tyr Ile Gln Ser Gln
            20                  25                  30

Tyr Gly Trp Arg Tyr Leu Ser Gly Gln Gln Glu Arg Ala Glu Val Gln
        35                  40                  45

Trp Leu Lys Ser Asn Ile Gly Lys Thr Pro Ala Ile Gln Gly Ser Asp
    50                  55                  60

Leu Ile Asp Tyr Ser Pro Ser Arg Val Ser Tyr Gly Ala Thr Ser Thr
65                  70                  75                  80

Ala Val Glu Asp Ala Ile Ala Phe Asp Arg Gln Gly Gly Ile Val Thr
                85                  90                  95

Phe Thr Trp His Trp Asn Ala Pro Asn Cys Leu Tyr Asn Ser Ala Asp
            100                 105                 110

Gln Pro Trp Tyr Phe Gly Phe Tyr Thr Lys Ala Thr Cys Phe Asn Ile
        115                 120                 125

Gln Ala Ala Leu Ala Gln Gly Ser Asn Gly Ala Asp Tyr Lys Leu Leu
    130                 135                 140

Ile Arg Asp Ile Asp Ala Ile Ala Val Gln Leu Lys Arg Leu Arg Asp
145                 150                 155                 160

Ala Lys Val Pro Ile Leu Phe Arg Pro Leu His Glu Pro Asp Gly Ala
                165                 170                 175

Trp Phe Trp Trp Gly Ala Lys Gly Ser Gly Pro Phe Lys Gln Leu Trp
            180                 185                 190

Asp Ile Leu Tyr Asp Arg Leu Thr Lys Tyr His Gly Leu His Asn Met
        195                 200                 205

Leu Trp Val Cys Asn Thr Glu Lys Ser Asp Trp Tyr Pro Gly Asn Asn
    210                 215                 220

Lys Cys Asp Ile Ala Thr Thr Asp Val Tyr Val Asn Ala Gly Asp His
225                 230                 235                 240

Ser Val Gln Lys Ser His Trp Asp Ala Leu Tyr Gly Val Ser Gly Gly
                245                 250                 255
```

```
Gln Arg Ile Leu Ala Leu Gly Glu Val Gly Val Ile Pro Asp Pro Glu
            260                 265                 270

Arg Gln Ala Ser Glu Asn Val Pro Trp Ala Tyr Trp Met Thr Trp Asn
        275                 280                 285

Gly Tyr Phe Ile Arg Asp Gly Asn Tyr Asn Ser Arg Asn Phe Leu Gln
    290                 295                 300

Ser Thr Phe Ser Asn Ala Arg Val Val Thr Leu Asp Gly Thr Ser Pro
305                 310                 315                 320

Leu Gly Asn Trp Lys Ser Ser
                325
```

## Description

REFERENCE TO A SEQUENCE LISTING

This application contains a Sequence Listing in computer readable form, which is incorporated herein by reference.

FIELD OF THE INVENTION

The present invention relates to cleaning compositions that include a galactanase enzyme, a peroxygen source; and an acyl hydrazone bleach catalyst. The present invention also relates to methods of making and using such cleaning compositions. The present invention also relates to the use of a galactanase enzyme, a peroxygen source and an acyl hydrazone bleach catalyst.

BACKGROUND OF THE INVENTION

The detergent formulator is constantly aiming to improve the performance of detergent compositions. One particular challenge is the removal of certain malodorous soils from surfaces such as textiles, especially under more sustainable washing processes involving cool wash temperatures.

There is a need for improved cleaning compositions that provide soil and/or malodor removal benefits.

SUMMARY OF THE INVENTION

The present invention provides a cleaning composition comprising an endo-beta-1,6-galactanase enzyme; a peroxygen source; and an acyl hydrazone bleach catalyst. The acyl hydrazone bleach catalyst may have a structure according to Formula I:

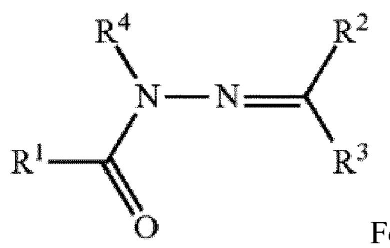

Formula I,

where $R^1$ stands for a $CF_3$ or for a $C_{1-28}$ alkyl, $C_{2-28}$ alkenyl, $C_{2-22}$ alkynyl, $C_{3-12}$ cycloalkyl, $C_{3-12}$ cycloalkenyl, phenyl, naphthyl, $C_{7-9}$ aralkyl, $C_{3-20}$ heteroalkyl or $C_{3-12}$ cycloheteroalkyl group,

$R^2$ and $R^3$ independently of one another stand for hydrogen or an optionally substituted $C_{1-28}$ alkyl, $C_{2-28}$ alkenyl, $C_{2-22}$ alkynyl, $C_{3-12}$ cycloalkyl, $C_{3-12}$ cycloalkenyl, $C_{7-9}$ aralkyl, $C_{3-28}$ heteroalkyl, $C_{3-12}$ cycloheteroalkyl, $C_{5-16}$ heteroaralkyl, phenyl, naphthyl or heteroaryl group or $R^2$ and $R^3$ together with the carbon atom linking them stand for an optionally substituted 5-, 6-, 7-, 8- or 9-membered ring that can optionally include heteroatoms, and

$R^4$ stands for hydrogen or a $C_{1-28}$ alkyl, $C_{2-28}$ alkenyl, $C_{2-22}$ alkynyl, $C_{3-12}$ cycloalkyl, $C_{3-12}$ cycloalkenyl, $C_{7-9}$ aralkyl, $C_{3-20}$ heteroalkyl, $C_{3-12}$ cycloheteroalkyl, $C_{5-16}$ heteroaralkyl group or an optionally substituted phenyl or naphthyl or heteroaryl group.

The present invention also provides a method of cleaning a surface, preferably a textile, where the method includes mixing a cleaning composition as described herein with water to form an aqueous liquor and contacting a surface, preferably a textile, with the aqueous liquor in a laundering step.

The present invention also relates to the use of an endo-beta-1,6-galactanase enzyme, a peroxygen source and an acyl hydrazone bleach catalyst in a cleaning composition to enhance stain-removal, bleaching, and/or malodor-reducing benefits.

DETAILED DESCRIPTION OF THE INVENTION

The present invention relates to cleaning compositions comprising a galactanase enzyme, a peroxygen source, and an acyl hydrazone bleach catalyst. Without wishing to be bound by theory, it is believed that the combination of acyl hydrazone bleach catalysts in combination with a peroxygen source synergistically interacts with a galactanase enzyme's

activity on soils, which may include activity on soils of microbial origin to improve soil and/or malodor removal. This might be due to the complimentary action of the acyl hydrazone bleach catalyst decolourising certain soil and malodor species, with the action of the galactanase enzyme in breaking down extracellular DNA soils which may play a role in adhering these soil and malodor components to textile surfaces. Certain galactanase enzymes may also be surprisingly robust in the presence of the bleach catalyst.

The components of the compositions and processes of the present invention are described in more detail below.

As used herein, the articles "a" and "an" when used in a claim, are understood to mean one or more of what is claimed or described. As used herein, the terms "include," "includes," and "including" are meant to be non-limiting. The compositions of the present invention can comprise, consist essentially of, or consist of, the components of the present invention.

The terms "substantially free of" or "substantially free from" may be used herein. This means that the indicated material is at the very minimum not deliberately added to the composition to form part of it, or, preferably, is not present at analytically detectable levels. It is meant to include compositions whereby the indicated material is present only as an impurity in one of the other materials deliberately included. The indicated material may be present, if at all, at a level of less than 1%, or less than 0.1 %, or less than 0.01 %, or even 0%, by weight of the composition.

Unless otherwise noted, all component or composition levels are in reference to the active portion of that component or composition, and are exclusive of impurities, for example, residual solvents or by-products, which may be present in commercially available sources of such components or compositions.

All temperatures herein are in degrees Celsius (°C) unless otherwise indicated. Unless otherwise specified, all measurements herein are conducted at 20°C and under the atmospheric pressure.

In all embodiments of the present invention, all percentages are by weight of the total composition, unless specifically stated otherwise. All ratios are weight ratios, unless specifically stated otherwise.

It should be understood that every maximum numerical limitation given throughout this specification includes every lower numerical limitation, as if such lower numerical limitations were expressly written herein. Every minimum numerical limitation given throughout this specification will include every higher numerical limitation, as if such higher numerical limitations were expressly written herein. Every numerical range given throughout this specification will include every narrower numerical range that falls within such broader numerical range, as if such narrower numerical ranges were all expressly written herein.

As used herein, the term "alkoxy" is intended to include C1-C8 alkoxy and C1-C8 alkoxy derivatives of polyols having repeating units such as butylene oxide, glycidol oxide, ethylene oxide or propylene oxide.

As used herein, unless otherwise specified, the terms "alkyl" and "alkyl capped" are intended to include C1-C18 alkyl groups, or even C1-C6 alkyl groups.

As used herein, unless otherwise specified, the term "aryl" is intended to include C3-12 aryl groups.

As used herein, unless otherwise specified, the term "arylalkyl" and "alkaryl" are equivalent and are each intended to include groups comprising an alkyl moiety bound to an aromatic moiety, typically having C1-C18 alkyl groups and, in one aspect, C1-C6 alkyl groups.

The terms "ethylene oxide," "propylene oxide" and "butylene oxide" may be shown herein by their typical designation of "EO," "PO" and "BO," respectively.

As used herein, the term "cleaning and/or treatment composition" includes, unless otherwise indicated, granular, powder, liquid, gel, paste, unit dose, bar form and/or flake type washing agents and/or fabric treatment compositions.

As used herein, "cellulosic substrates" are intended to include any substrate which comprises cellulose, either 100% by weight cellulose or at least 20% by weight, or at least 30 % by weight or at least 40 or at least 50 % by weight or even at least 60 % by weight cellulose. Cellulose may be found in wood, cotton, linen, jute, and hemp. Cellulosic substrates may be in the form of powders, fibers, pulp and articles formed from powders, fibers and pulp. Cellulosic fibers, include, without limitation, cotton, rayon (regenerated cellulose), acetate (cellulose acetate), triacetate (cellulose triacetate), and mixtures thereof. Typically cellulosic substrates comprise cotton. Articles formed from cellulosic fibers include textile articles such as fabrics. Articles formed from pulp include paper.

As used herein, the term "maximum extinction coefficient" is intended to describe the molar extinction coefficient at the wavelength of maximum absorption (also referred to herein as the maximum wavelength), in the range of 400 nanometers to 750 nanometers.

As used herein "average molecular weight" is reported as a weight average molecular weight, as determined by its molecular weight distribution; as a consequence of their manufacturing process, polymers disclosed herein may contain a distribution of repeating units in their polymeric moiety.

As used herein the term "variant" refers to a polypeptide that contains an amino acid sequence that differs from a wild type or reference sequence. A variant polypeptide can differ from the wild type or reference sequence due to a deletion, insertion, or substitution of a nucleotide(s) relative to said reference or wild type nucleotide sequence. The reference or wild type sequence can be a full-length native polypeptide sequence or any other fragment of a full- length polypeptide sequence. A polypeptide variant generally has at least about 70% amino acid sequence identity with the

reference sequence, but may include 75% amino acid sequence identity within the reference sequence, 80% amino acid sequence identity within the reference sequence, 85% amino acid sequence identity with the reference sequence, 86% amino acid sequence identity with the reference sequence, 87% amino acid sequence identity with the reference sequence, 88% amino acid sequence identity with the reference sequence, 89% amino acid sequence identity with the reference sequence, 90% amino acid sequence identity with the reference sequence, 91% amino acid sequence identity with the reference sequence, 92% amino acid sequence identity with the reference sequence, 93% amino acid sequence identity with the reference sequence, 94% amino acid sequence identity with the reference sequence, 95% amino acid sequence identity with the reference sequence, 96% amino acid sequence identity with the reference sequence, 97% amino acid sequence identity with the reference sequence, 98% amino acid sequence identity with the reference sequence, 98.5% amino acid sequence identity with the reference sequence or 99% amino acid sequence identity with the reference sequence.

As used herein, the term "solid" includes granular, powder, bar and tablet product forms.

As used herein, the term "fluid" includes liquid, gel, paste, and gas product forms.

## Cleaning Composition

The present disclosure relates to cleaning and/or treatment compositions. The cleaning composition may be selected from the group of light duty liquid detergents compositions, heavy duty liquid detergent compositions, solid, for example particulate/powder or "dry" cleaning compositions, hard surface cleaning compositions, detergent gels commonly used for laundry, bleaching compositions, laundry additives, fabric enhancer compositions, shampoos, body washes, other personal care compositions, and mixtures thereof. The cleaning composition may be a hard surface cleaning composition (such as a dishwashing composition) or a laundry composition (such as a heavy duty liquid or solid detergent composition).

The cleaning compositions may be in any suitable form. The composition can be selected from a liquid, solid, or combination thereof. As used herein, "liquid" includes free-flowing liquids, as well as pastes, gels, foams and mousses. Non-limiting examples of liquids include light duty and heavy duty liquid detergent compositions, fabric enhancers, detergent gels commonly used for laundry, bleach and laundry additives. Gases, e.g., suspended bubbles, or solids, e.g. particles, may be included within the liquids. A "solid" as used herein includes, but is not limited to, powders, agglomerates, and mixtures thereof. Non-limiting examples of solids include: granules, micro-capsules, beads, noodles, and pearlised balls. Solid compositions may provide a technical benefit including, but not limited to, through-the-wash benefits, pre-treatment benefits, and/or aesthetic effects.

The cleaning composition may be in the form of a unitized dose article, such as a tablet or in the form of a pouch. Such pouches typically include a water-soluble film, such as a polyvinyl alcohol water-soluble film, that at least partially encapsulates a composition. Suitable films are available from MonoSol, LLC (Indiana, USA). The composition can be encapsulated in a single or multi-compartment pouch. A multi-compartment pouch may have at least two, at least three, or at least four compartments. A multi-compartmented pouch may include compartments that are side-by-side and/or superposed. The composition contained in the pouch may be liquid, solid (such as powders), or combinations thereof.

## Galactanase Enzyme

The endo-beta-1,6-galactanase enzyme is an extracellular polymer-degrading enzyme. The term "endo-beta-1,6-galactanase" or "a polypeptide having endo-beta-1,6-galactanase activity" means a endo-beta-1,6-galactanase activity (EC 3.2.1.164) that catalyzes the hydrolytic cleavage of 1,6-3-D-galactooligosaccharides with a degree of polymerization (DP) higher than 3, and their acidic derivatives with 4-O-methylglucosyluronate or glucosyluronate groups at the non-reducing terminals.

For purposes of the present disclosure, endo-beta-1,6-galactanase activity is determined according to the procedure described in WO 2015185689 in Assay I. Suitable examples from class EC 3.2.1.164 are described in WO 2015185689, such as the mature polypeptide SEQ ID NO: 2 described therein. Preferably the galactanase enzyme is selected from Glycoside Hydrolase (GH) Family 30.

Preferably, the endo-beta-1,6-galactanase comprises a microbial enzyme. The endo-beta-1,6-galactanase may be fungal or bacterial in origin. Bacterial endo-beta-1,6-galactanase may be most preferred. Fungal endo-beta-1,6-galactanase may be most preferred.

A bacterial endo-beta-1,6-galactanase is obtainable from *Streptomyces,* for example *Streptomyces davawensis.* A preferred endo-beta-1,6-galactanase is obtainable from *Streptomyces davawensis* JCM 4913 defined in SEQ ID NO: 1 herein, or a variant thereof, for example having at least 40% or 50% or 60% or 70% or 75% or 80% or 85% or 90% or 95%, 96%, 97%, 98%, 99% or 100% identity thereto.

Other bacterial endo-beta-1,6-galactanase include those encoded by the DNA sequences of *Streptomyces*

*avermitilis* MA-4680 with amino acid sequence defined in SEQ ID NO: 2 herein, or a variant thereof, for example having at least 40% or 50% or 60% or 70% or 75% or 80% or 85% or 90% or 95%, 96%, 97%, 98%, 99% or 100% identity thereto.

A fungal endo-beta-1,6-galactanase is obtainable from *Trichoderma,* for example *Trichoderma harzianum.* A preferred endo-beta-1,6-galactanase is obtainable from *Trichoderma harzianum* defined in SEQ ID NO: 3 herein, or a variant thereof, for example having at least 40% or 50% or 60% or 70% or 75% or 80% or 85% or 90% or 95%, 96%, 97%, 98%, 99% or 100% identical thereto.

Other fungal endo-beta-1,6-galactanases include those encoded by the DNA sequences of *Ceratocystis fimbriata* f. sp. Platani, *Muscodor strobelii* WG-2009a, *Oculimacula yallundae, Trichoderma viride* GD36A, *Thermomyces stellatus, Myceliophthora thermophilia.*

Preferably the galactanase has an amino acid sequence having at least 60%, or at least 80%, or at least 90% or at least 95% identity with the amino acid sequence shown in SEQ ID NO:1, SEQ ID NO:2 or SEQ ID NO:3. Preferably the galactanase is an isolated galactanase.

Preferably the galactanase enzyme is present in a laundering aqueous liquor in an amount of from 0.01ppm to 1000 ppm of the galactanase enzyme, or from 0.05 or from 0.1ppm to 750 or 500ppm.

The galactanase or composition comprising galactanase may also give rise to biofilm-disrupting effects.

Peroxygen Source

The cleaning compositions of the present invention comprise a peroxygen source. Peroxygen sources are known to provide, for example, bleaching benefits. The peroxygen source may be present in the cleaning composition at a level of from about 0.1% to about 40%, or from about 0.5% to about 20%, by weight of the cleaning composition.

The peroxygen source may be $H_2O_2$ or a substance that releases $H_2O_2$ in water. The peroxygen source may be selected from the group consisting of perborates, percarbonates, perhydrates, peroxycarboxylic acids, persulfates, preoxydisulfates, diacyl peroxides, tetraacyl diperoxides, and combinations thereof. The peroxygen source may be selected from the group consisting of alkali metal perborates, alkali metal percarbonates, urea perhydrates, and combination thereof. The peroxygen source may comprises a peroxycarboxylic acid selected from diperoxydecanedicarboxylic acid, phthalimido peroxycaproic acid, or combinations thereof.

Acyl hydrazone bleach catalyst

The cleaning compositions of the present invention include an acyl hydrazone bleach catalyst. The acyl hydrazone bleach catalyst may be present in the cleaning composition at a level of from about 0.005% to about 1%,, or from about 0.01% to about 0.5%, by weight of the cleaning composition.

The acyl hydrazone bleach catalyst may have a structure according to the general Formula I:

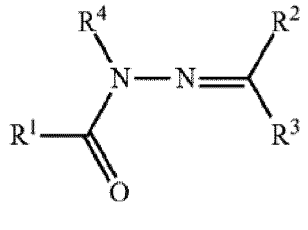

Formula I

in which $R^1$ stands for a $CF_3$ or for a $C_{1-28}$ alkyl, $C_{2-28}$ alkenyl, $C_{2-22}$ alkynyl, $C_{3-12}$ cycloalkyl, $C_{3-12}$ cycloalkenyl, phenyl, naphthyl, $C_{7-9}$ aralkyl, $C_{3-20}$ heteroalkyl or $C_{3-12}$ cycloheteroalkyl group, $R^2$ and $R^3$ independently of one another stand for hydrogen or an optionally substituted $C_{1-28}$ alkyl, $C_{2-28}$ alkenyl, $C_{2-22}$ alkynyl, $C_{3-12}$ cycloalkyl, $C_{3-12}$ cycloalkenyl, $C_{7-9}$ aralkyl, $C_{3-28}$ heteroalkyl, $C_{3-12}$ cycloheteroalkyl, $C_{5-16}$ heteroaralkyl, phenyl, naphthyl or heteroaryl group or $R^2$ and $R^3$ together with the carbon atom linking them stand for an optionally substituted 5-, 6-, 7-, 8- or 9-membered ring that can optionally comprise heteroatoms, and $R^4$ stands for hydrogen or a $C_{1-28}$ alkyl, $C_{2-28}$ alkenyl, $C_{2-22}$ alkynyl, $C_{3-12}$ cycloalkyl, $C_{3-12}$ cycloalkenyl, $C_{7-9}$ aralkyl, $C_{3-20}$ heteroalkyl, $C_{3-12}$ cycloheteroalkyl, $C_{5-16}$ heteroaralkyl group or an optionally substituted phenyl or naphthyl or heteroaryl group.

The acyl hydrazone bleach catalyst may have a structure according to Formula I, wherein $R^1$ stands for a $C_{3-12}$ cycloheteroalkyl group, $R^2$ and $R^3$ independently of one another stand for hydrogen or an optionally substituted phenyl group, and $R^4$ stands for hydrogen.

One particularly preferred acyl hydrazone bleach catalyst is 4-(2-(2-((2-hydroxyphenylmethyl)methylene)-hydrazinyl)-2-oxoethyl)-4-methylchloride, shown in Formula 2 below.

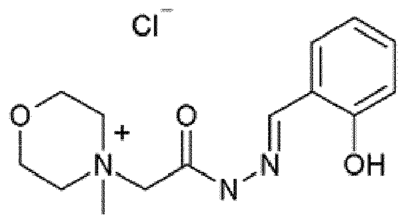

Formula II

Without wishing to be bound by theory, the acyl hydrazone bleach catalysts described herein boost the bleaching action of peroxidic bleaching agents, without unduly damaging the substrate to be cleaned, for example the fabric. The peroxidic bleaching agents are preferably $H_2O_2$ or substances that release $H_2O_2$ in water, including in particular alkali metal perborates, alkali metal percarbonates and urea perhydrates; however, they may be also possibly employed combined with peroxycarboxylic acids, such as diperoxydecanedicarboxylic acid or phthalimido peroxycaproic acid, with other acids or acidic salts, such as alkali metal persulfates or alkali metal peroxydisulfates or Caroates, or with diacyl peroxides or tetraacyl diperoxides.

Adjuncts

The cleaning compositions described herein may optionally include other adjunct components, for example selected from surfactants, fabric shading dyes, fabric care benefit agent; additional enzyme; deposition aid; rheology modifier; builder; chelant; bleach; bleaching agent; bleach precursor; bleach booster; bleach activator, bleach catalyst; perfume and/or perfume microcapsules; perfume loaded zeolite; starch encapsulated accord; polyglycerol esters; whitening agent; pearlescent agent; enzyme stabilizing systems; scavenging agents including fixing agents for anionic dyes, complexing agents for anionic surfactants, and mixtures thereof; optical brighteners or fluorescers; polymer including but not limited to soil release polymer and/or soil suspension polymer; dispersants; antifoam agents; non-aqueous solvent; fatty acid; suds suppressors, e.g., silicone suds suppressors; cationic starches; scum dispersants; substantive dyes; colorants; opacifier; antioxidant; hydrotropes such as toluenesulfonates, cumenesulfonates and naphthalenesulfonates; color speckles; colored beads, spheres or extrudates; clay softening agents; anti-bacterial agents. Additionally or alternatively, the compositions may comprise surfactants, and/or solvent systems. Quaternary ammonium compounds may be present, particularly in fabric enhancer compositions, such as fabric softeners, and comprise quaternary ammonium cations that are positively charged polyatomic ions of the structure $NR_4^+$, where R is an alkyl group or an aryl group.

Additional Enzymes

Preferably the composition of the invention comprises additional enzymes, for example selected from lipases, amylases, proteases, nucleases, pectate lyases, cellulases, cutinases, and mixtures thereof. The cleaning compositions preferably comprise one or more additional enzymes from the group selected from nucleases. The cleaning compositions preferably comprises one or more additional enzymes selected from the group amylases, lipases, proteases, pectate lyases, cellulases, cutinases, and mixtures thereof. Preferably, the cleaning compositions comprises one or more additional enzymes selected from amylases and proteases and mixtures thereof. Preferably the cleaning compositions comprise one or more additional enzymes selected from lipases. The compositions may also comprise hemicellulases, peroxidases, xylanases, pectinases, keratinases, reductases, oxidases, phenoloxidases, lipoxygenases, ligninases, pullulanases, tannases, pentosanases, malanases, ß-glucanases, arabinosidases, hyaluronidase, chondroitinase, laccase and mixtures thereof. When present in the composition, the aforementioned additional enzymes may be present at levels from about 0.00001% to about 2%, from about 0.0001 % to about 1% or even from about 0.001 % to about 0.5% enzyme protein by weight of the composition. Preferably the or each additional enzyme is present in the laundering aqueous liquor in an amount of from 0.01ppm to 1000 ppm of the active enzyme protein, or from 0.05 or from 0.1ppm to 750 or 500ppm.

Nucleases

Preferably the composition additionally comprises a nuclease enzyme. The nuclease enzyme is an enzyme capable of cleaving the phosphodiester bonds between the nucleotide subunits of nucleic acids. Suitable nuclease enzymes may be deoxyribonuclease or ribonuclease enzyme or a functional fragment thereof. By functional fragment

or part is meant the portion of the nuclease enzyme that catalyzes the cleavage of phosphodiester linkages in the DNA backbone and so is a region of said nuclease protein that retains catalytic activity. Thus it includes truncated, but functional versions, of the enzyme and/or variants and/or derivatives and/or homologues whose functionality is maintained.

Preferably the nuclease enzyme is a deoxyribonuclease, preferably selected from any of the classes E.C. 3.1.21.x, where x=1, 2, 3, 4, 5, 6, 7, 8 or 9, E.C. 3.1.22.y where y=1, 2, 4 or 5, E.C. 3.1.30.z where z= 1 or 2, E.C. 3.1.31.1 and mixtures thereof. Nuclease enzymes from class E.C. 3.1.21.x and especially where x=1 are particularly preferred. Nucleases in class E.C. 3.1.22.y cleave at the 5' hydroxyl to liberate 3' phosphomonoesters. Enzymes in class E.C. 3.1.30.z may be preferred as they act on both DNA and RNA and liberate 5'-phosphomonoesters. Suitable examples from class E.C. 3.1.31.2 are described in US2012/0135498A, such as SEQ ID NO:3 therein. Such enzymes are commercially available as DENARASE® enzyme from c-LECTA. Nuclease enzymes from class E.C. 3.1.31.1 produce 3'phosphomonoesters.

Preferably, the nuclease enzyme comprises a microbial enzyme. The nuclease enzyme may be fungal or bacterial in origin. Bacterial nucleases may be most preferred. Fungal nucleases may be most preferred.

The microbial nuclease is obtainable from *Bacillus,* such as a *Bacillus licheniformis* or *Bacillus subtilis* bacterial nucleases. A preferred nuclease is obtainable from *Bacillus licheniformis,* preferably from strain EI-34-6. A preferred deoxyribonuclease is a variant of *Bacillus licheniformis,* from strain EI-34-6 nucB deoxyribonuclease defined in SEQ ID NO:4 herein, or variant thereof, for example having at least 70% or 75% or 80% or 85% or 90% or 95%, 96%, 97%, 98%, 99% or 100% identical thereto. Other suitable nucleases are defined in SEQ ID NO: 5 herein, or variant thereof, for example having at least 70% or 75% or 80% or 85% or 90% or 95%, 96%, 97%, 98%, 99% or 100% identical thereto. Other suitable nucleases are defined in SEQ ID NO: 6 herein, or variant thereof, for example having at least 70% or 75% or 80% or 85% or 90% or 95%, 96%, 97%, 98%, 99% or 100% identical thereto.

A fungal nuclease is obtainable from *Aspergillus,* for example *Aspergillus oryzae.* A preferred nuclease is obtainable from *Aspergillus oryzae* defined in SEQ ID NO: 7 herein, or variant thereof, for example having at least 60% or 70% or 75% or 80% or 85% or 90% or 95%, 96%, 97%, 98%, 99% or 100% identical thereto.

Another suitable fungal nuclease is obtainable from *Trichoderma,* for example *Trichoderma harzianum.* A preferred nuclease is obtainable from *Trichoderma harzianum* defined in SEQ ID NO: 8 herein, or variant thereof, for example having at least 60% or 70% or 75% or 80% or 85% or 90% or 95%, 96%, 97%, 98%, 99% or 100% identical thereto.

Other fungal nucleases include those encoded by the DNA sequences of *Aspergillus oryzae* RIB40, *Aspergillus oryzae* 3.042, *Aspergillus flavus* NRRL3357, *Aspergillus parasiticus* SU-1, *Aspergillus nomius* NRRL13137, *Trichoderma reesei* QM6a, *Trichoderma virens* Gv29-8, *Oidiodendron maius* Zn, *Metarhizium guizhouense* ARSEF 977, *Metarhizium majus* ARSEF *297, Metarhizium robertsii* ARSEF 23, *Metarhizium acridum* CQMa 102, *Metarhizium brunneum* ARSEF 3297, *Metarhizium anisopliae, Colletotrichum fioriniae* PJ7, *Colletotrichum sublineola, Trichoderma atroviride* IMI 206040, *Tolypocladium ophioglossoides* CBS 100239, *Beauveria bassiana* ARSEF 2860, *Colletotrichum higginsianum, Hirsutella minnesotensis* 3608, *Scedosporium apiospermum, Phaeomoniella chlamydospora, Fusarium verticillioides* 7600, *Fusarium oxysporum* f. sp. cubense race 4, *Colletotrichum graminicola* M1.001, *Fusarium oxysporum* FOSC 3-a, *Fusarium avenaceum, Fusarium langsethiae, Grosmannia clavigera* kw1407, *Claviceps purpurea* 20.1, *Verticillium longisporum, Fusarium oxysporum* f. sp. cubense race 1, *Magnaporthe oryzae* 70-15, *Beauveria bassiana* D1-5, *Fusarium pseudograminearum* CS3096, *Neonectria ditissima, Magnaporthiopsis poae* ATCC 64411, *Cordyceps militaris* CM01, *Marssonina brunnea* f. sp. 'multigermtubi' MB_m1, *Diaporthe ampelina, Metarhizium album* ARSEF 1941, *Colletotrichum gloeosporioides* Nara gc5, *Madurella mycetomatis, Metarhizium brunneum* ARSEF 3297, *Verticillium alfalfae* VaMs.102, *Gaeumannomyces graminis* var. tritici R3-111a-1, *Nectria haematococca* mpVI 77-13-4, *Verticillium longisporum, Verticillium dahliae* VdLs.17, *Torrubiella hemipterigena, Verticillium longisporum, Verticillium dahliae* VdLs.17, *Botrytis cinerea* B05.10, *Chaetomium globosum* CBS 148.51, *Metarhizium anisopliae, Stemphylium lycopersici, Sclerotinia borealis* F-4157, *Metarhizium robertsii* ARSEF *23, Myceliophthora thermophila* ATCC 42464, *Phaeosphaeria nodorum* SN15, *Phialophora attae, Ustilaginoidea virens, Diplodia seriata, Ophiostoma piceae* UAMH 11346, *Pseudogymnoascus pannorum* VKM F-4515 (FW-2607), *Bipolaris oryzae* ATCC 44560, *Metarhizium guizhouense* ARSEF 977, *Chaetomium thermophilum* var. thermophilum DSM 1495, *Pestalotiopsis fici* W106-1, *Bipolaris zeicola* 26-R-13, *Setosphaeria turcica* Et28A, *Arthroderma otae* CBS 113480 and *Pyrenophora tritici-repentis* Pt-1C-BFP.

Preferably the nuclease is an isolated nuclease.

Preferably the nuclease enzyme is present in the laundering aqueous liquor in an amount of from 0.01ppm to 1000 ppm of the nuclease enzyme, or from 0.05 or from 0.1ppm to 750 or 500ppm.

Acetylglucosaminidases.

Preferably the composition comprises an acetylglucosaminidase enzyme, preferably a β-N-acetylglucosaminidase enzyme from E.C. 3.2.1.52, preferably an enzyme having at least 70%, or at least 75% or at least 80% or at least 85% or at least 90% or at least 95% or at least 96% or at least 97% or at least 98% or at least 99% or at least or 100% identity to SEQ ID NO: 9.

Mannanases

Preferably the composition comprises a mannanase enzyme. The term "mannanase" means a polypeptide having mannan endo-1,4-beta-mannosidase activity (EC 3.2.1.78) from the glycoside hydrolase family 26 that catalyzes the hydrolysis of 1 ,4-3-D-mannosidic linkages in mannans, galactomannans and glucomannans. Alternative names of mannan endo-1,4-beta-mannosidase are 1,4-3-D-mannan mannanohydrolase; endo-1,4-3-mannanase; endo-β-1,4-mannase; β-mannanase B; 3-1,4-mannan 4-mannanohydrolase; endo-3-mannanase; and β-D-mannanase. Preferred mannanases are members of the glycoside hydrolase family 26.

For purposes of the present disclosure, mannanase activity may be determined using the Reducing End Assay as described in the experimental section of WO 2015040159.

Suitable examples from class EC 3.2.1.78 are described in WO 2015040159, such as the mature polypeptide SEQ ID NO: 2 described therein.

Preferred mannanases are variants having at least 60%, at least 65%, at least 70%, at least 75%, at least 80%, at least 81 %, at least 82%, at least 83%, at least 84%, at least 85%, at least 86%, at least 87%, at least 88%, at least 89%, at least 90%, at least 91 %, at least 92%, at least 93%, at least 94%, at least 95%, at least 96%, at least 97%, at least 98%, at least 99% or 100% sequence identity to the mature polypeptide SEQ ID NO: 10 from *Ascobolus stictoideus;*

Preferred mannanases are variants having at least 81 %, at least 82%, at least 83%, at least 84%, at least 85%, at least 86%, at least 87%, at least 88%, at least 89%, at least 90%, at least 91 %, at least 92%, at least 93%, at least 94%, at least 95%, at least 96%, at least 97%, at least 98%, at least 99% or 100% sequence identity to the mature polypeptide SEQ ID NO: 11 from *Chaetomium virescens.*

Preferred mannanases are variants having at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91 %, at least 92%, at least 93%, at least 94%, at least 95%, at least 96%, at least 97%, at least 98%, at least 99% or 100% sequence identity to the mature polypeptide SEQ ID NO: 12 from *Preussia aemulans.*

Preferred mannanases are variants having at least at least 65%, at least 66%, at least 67%, at least 68%, at least 69%, at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91 %, at least 92%, at least 93%, at least 94%, at least 95%, at least 96%, at least 97%, at least 98%, at least 99% or 100% sequence identity to the mature polypeptide SEQ ID NO: 13 from *Yunnania penicillata.*

Preferred mannanases are variants having at least at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91 %, at least 92%, at least 93%, at least 94%, at least 95%, at least 96%, at least 97%, at least 98%, at least 99% or 100% sequence identity to the mature polypeptide SEQ ID NO: 14 from *Myrothecium roridum.* Preferably the mannanase is an isolated mannanase.

Preferably the mannanase enzyme is present in the cleaning compositions in an amount from 0.001 to 1 wt% based on active protein in the composition, or from 0.005 to 0.5 wt% or from 0.01 to 0.25 wt%. Preferably the mannanase enzyme is present in the laundering aqueous liquor in an amount of from 0.01ppm to 1000 ppm of the mannanase enzyme, or from 0.05 or from 0.1ppm to 750 or 500ppm. The compositions of the invention comprising both galactanase and mannanase may be particularly effective against sticky soils and for improved cleaning. It is believed the two enzymes function together in a complementary way.

Further Glycosyl Hydrolases

The composition may comprise a glycosyl hydrolase selected from GH family 39 and GH family 114 and mixtures thereof, for example as described in co-pending applications having applicants reference numbers CM4645FM and CM4646 FM, respectively.

Proteases.

Preferably the composition comprises one or more proteases. Suitable proteases include metalloproteases and serine proteases, including neutral or alkaline microbial serine proteases, such as subtilisins (EC 3.4.21.62). Suitable proteases include those of animal, vegetable or microbial origin. In one aspect, such suitable protease may be of microbial origin. The suitable proteases include chemically or genetically modified mutants of the aforementioned suitable proteases. In one aspect, the suitable protease may be a serine protease, such as an alkaline microbial protease or/and a trypsin-type protease. Examples of suitable neutral or alkaline proteases include:

(a) subtilisins (EC 3.4.21.62), preferably those derived from *Bacillus sp.,* such as *B. lentus, B. alkalophilus, B. subtilis, B. amyloliquefaciens, B. pumilus* and *B.* gibsonii and *B. akibaii* described in WO2004067737, WO2015091989, WO2015091990, WO2015024739, WO2015143360, US 6,312,936 B1, US 5,679,630, US 4,760,025, US7,262,042 and WO09/021867, DE102006022216A1, DE102006022224A1, WO2015089447, WO2015089441, WO2016066756, WO2016066757, WO2016069557, WO2016069563, WO2016069569.

(b) trypsin-type or chymotrypsin-type proteases, such as trypsin (e.g., of porcine or bovine origin), including the *Fusarium* protease described in WO 89/06270 and the chymotrypsin proteases derived from *Cellumonas* described in WO 05/052161 and WO 05/052146.

(c) metalloproteases, preferably those derived from *Bacillus amyloliquefaciens* described in WO 07/044993A2; from *Bacillus, Brevibacillus, Thermoactinomyces, Geobacillus, Paenibacillus, Lysinibacillus* or *Streptomyces spp.* Described in WO2014194032, WO2014194054 and WO2014194117; from *Kribella alluminosa* described in WO2015193488; and from *Streptomyces* and *Lysobacter* described in WO2016075078.

(d) protease having at least 90% identity to the subtilase from Bacillus sp. TY145, NCIMB 40339, described in WO92/17577 (Novozymes A/S), including the variants of this Bacillus sp TY145 subtilase described in WO2015024739, and WO2016066757.

Preferred proteases include those derived from *Bacillus gibsonii* or *Bacillus Lentus.*

Suitable commercially available protease enzymes include those sold under the trade names Alcalase®, Savinase®, Primase®, Durazym®, Polarzyme®, Kannase®, Liquanase®, Liquanase Ultra®, Savinase Ultra®, Ovozyme®, Neutrase®, Everlase® and Esperase® by Novozymes A/S (Denmark), those sold under the tradename Maxatase®, Maxacal®, Maxapem®, Properase®, Purafect®, Purafect Prime®, Purafect Ox®, FN3® , FN4®, Excellase® and Purafect OXP® by Genencor International, those sold under the tradename Opticlean® and Optimase® by Solvay Enzymes, those available from Henkel/ Kemira, namely BLAP (sequence shown in Figure 29 of US 5,352,604 with the following mutations S99D + S101 R + S103A + V104I + G159S, hereinafter referred to as BLAP), BLAP R (BLAP with S3T + V4I + V199M + V205I + L217D), BLAP X (BLAP with S3T + V4I + V205I) and BLAP F49 (BLAP with S3T + V4I + A194P + V199M + V205I + L217D) - all from Henkel/Kemira; and KAP (*Bacillus alkalophilus* subtilisin with mutations A230V + S256G + S259N) from Kao, or as disclosed in WO2009/149144, WO2009/149145, WO2010/56653, WO2010/56640, WO2011/072117, US2011/0237487, WO2011/140316, WO2012/151480, EP2510092, EP2566960 OR EP2705145.

Amylases

Preferably the composition may comprise an amylase. Suitable alpha-amylases include those of bacterial or fungal origin. Chemically or genetically modified mutants (variants) are included. A preferred alkaline alpha-amylase is derived from a strain of Bacillus, such as Bacillus licheniformis, Bacillus amyloliquefaciens, Bacillus stearothermophilus, Bacillus subtilis, or other Bacillus sp., such as Bacillus sp. NCIB 12289, NCIB 12512, NCIB 12513, DSM 9375 (USP 7,153,818) DSM 12368, DSMZ no. 12649, KSM AP1378 (WO 97/00324), KSM K36 or KSM K38 (EP 1,022,334). Preferred amylases include:

(a) the variants described in WO 94/02597, WO 94/18314, WO96/23874 and WO 97/43424, especially the variants with substitutions in one or more of the following positions versus the enzyme listed as SEQ ID No. 2 in WO 96/23874: 15, 23, 105, 106, 124, 128, 133, 154, 156, 181, 188, 190, 197, 202, 208, 209, 243, 264, 304, 305, 391, 408, and 444.

(b) the variants described in USP 5,856,164 and WO99/23211, WO 96/23873, WO00/60060 and WO 06/002643, especially the variants with one or more substitutions in the following positions versus the AA560 enzyme listed as SEQ ID No. 12 in WO 06/002643:

26, 30, 33, 82, 37, 106, 118, 128, 133, 149, 150, 160, 178, 182, 186, 193, 203, 214, 231, 256, 257, 258, 269, 270, 272, 283, 295, 296, 298, 299, 303, 304, 305, 311, 314, 315, 318, 319, 339, 345, 361, 378, 383, 419, 421, 437, 441, 444, 445, 446, 447, 450, 461, 471, 482, 484, preferably that also contain the deletions of D183* and G184*.

(c) variants exhibiting at least 90% identity with SEQ ID No. 4 in WO06/002643, the wild-type enzyme from Bacillus SP722, especially variants with deletions in the 183 and 184 positions and variants described in WO 00/60060, which is incorporated herein by reference.

(d) variants exhibiting at least 95% identity with the wild-type enzyme from Bacillus sp.707 (SEQ ID NO:7 in US 6,093, 562), especially those comprising one or more of the following mutations M202, M208, S255, R172, and/or M261. Preferably said amylase comprises one or more of M202L, M202V, M202S, M202T, M202I, M202Q, M202W, S255N and/or R172Q. Particularly preferred are those comprising the M202L or M202T mutations.

(e) variants described in WO 09/149130, preferably those exhibiting at least 90% identity with SEQ ID NO: 1 or SEQ

ID NO:2 in WO 09/149130, the wild-type enzyme from Geobacillus Stearophermophilus or a truncated version thereof;

(f) variants as described in EP2540825 and EP2357220, EP2534233; (g) variants as described in WO2009100102 and WO2010115028.

Suitable commercially available alpha-amylases include DURAMYL®, LIQUEZYME®, TERMAMYL®, TERMAMYL ULTRA®, NATALASE®, SUPRAMYL®, STAINZYME®, STAINZYME PLUS®, FUNGAMYL® and BAN® (Novozymes A/S, Bagsvaerd, Denmark), KEMZYM® AT 9000 Biozym Biotech Trading GmbH Wehlistrasse 27b A-1200 Wien Austria, RAPIDASE® , PURASTAR®, ENZYSIZE®, OPTISIZE HT PLUS®, POWERASE® and PURASTAR OXAM® (Genencor International Inc., Palo Alto, California) and KAM® (Kao, 14-10 Nihonbashi Kayabacho, 1-chome, Chuo-ku Tokyo 103-8210, Japan). In one aspect, suitable amylases include NATALASE®, STAINZYME® and STAINZYME PLUS® and mixtures thereof.

Lipases

Preferably the composition comprises one or more lipases, including "first cycle lipases" such as those described in U.S. Patent 6,939,702 B1 and US PA 2009/0217464. Preferred lipases are first-wash lipases. In one embodiment of the invention the composition comprises a first wash lipase. First wash lipases includes a lipase which is a polypeptide having an amino acid sequence which: (a) has at least 90% identity with the wild-type lipase derived from *Humicola lanuginosa* strain DSM 4109; (b) compared to said wild-type lipase, comprises a substitution of an electrically neutral or negatively charged amino acid at the surface of the three-dimensional structure within 15A of E1 or Q249 with a positively charged amino acid; and (c) comprises a peptide addition at the C-terminal; and/or (d) comprises a peptide addition at the N-terminal and/or (e) meets the following limitations: i) comprises a negative amino acid in position E210 of said wild-type lipase; ii) comprises a negatively charged amino acid in the region corresponding to positions 90-101 of said wild-type lipase; and iii) comprises a neutral or negative amino acid at a position corresponding to N94 or said wild-type lipase and/or has a negative or neutral net electric charge in the region corresponding to positions 90-101 of said wild-type lipase. Preferred are variants of the wild-type lipase from *Thermomyces lanuginosus* comprising one or more of the T231R and N233R mutations. The wild-type sequence is the 269 amino acids (amino acids 23 - 291) of the Swissprot accession number Swiss-Prot 059952 (derived from *Thermomyces lanuginosus* (*Humicola lanuginosa*)). Preferred lipases include those sold under the tradenames Lipex® and Lipolex® and Lipoclean®. Other suitable lipases include those described in European Patent Application No. 12001034.3 or EP2623586.

Endoglucanases

Other preferred enzymes include microbial-derived endoglucanases exhibiting endo-beta-1,4-glucanase activity (E.C. 3.2.1.4), including a bacterial polypeptide endogenous to a member of the genus Bacillus which has a sequence of at least 90%, 94%, 97% and even 99% identity to the amino acid sequence SEQ ID NO:2 in US7,141,403B2) and mixtures thereof. Suitable endoglucanases are sold under the tradenames Celluclean® and Whitezyme® (Novozymes A/S, Bagsvaerd, Denmark).

Pectate Lyases

Other preferred enzymes include pectate lyases sold under the tradenames Pectawash®, Pectaway®, Xpect® and mannanases sold under the tradenames Mannaway® (all from Novozymes A/S, Bagsvaerd, Denmark), and Purabrite® (Genencor International Inc., Palo Alto, California).

Cleaning Cellulase

The cleaning composition described herein may additionally comprise a cleaning cellulase. The cellulase may be an endoglucanase. The cellulase may have endo beta 1,4-glucanase activity and a structure which does not comprise a class A Carbohydrate Binding Module (CBM). A class A CBM is defined according to A. B. Boraston et al. Biochemical Journal 2004, Volume 382 (part 3) pages 769-781. In particular, the cellulase does not comprise a class A CBM from families 1, 2a, 3, 5 and 10.

The cellulase may be a glycosyl hydrolase having enzymatic activity towards amorphous cellulose substrates, wherein the glycosyl hydrolase is selected from GH families 5, 7, 12, 16, 44 or 74. Preferably, the cellulase is a glycosyl hydrolase selected from GH family 5. A preferred cellulase is Celluclean, supplied by Novozymes. This preferred cellulase is described in more detail in WO2002/099091. The glycosyl hydrolase (GH) family definition is described in more detail in Biochem J. 1991, v280, 309-316. Another preferred cellulase is a glycosyl hydrolase having enzymatic activity towards

both xyloglucan and amorphous cellulose substrates, wherein the glycosyl hydrolase is selected from GH families 5, 12, 44 or 74. Preferably, the glycosyl hydrolase selected from GH family 44.

For purposes of the present invention, the degree of identity between two amino acid sequences is determined using the Needleman-Wunsch algorithm (Needleman and Wunsch, 1970, J. Mol. Biol. 48: 443-453) as implemented in the Needle program of the EMBOSS package (EMBOSS: The European Molecular Biology Open Software Suite, Rice et al., 2000, Trends in Genetics 16: 276-277), preferably version 3.0.0 or later. The optional parameters used are gap open penalty of 10, gap extension penalty of 0.5, and the EBLOSUM62 (EMBOSS version of BLOSUM62) substitution matrix. The output of Needle labeled "longest identity" (obtained using the -nobrief option) is used as the percent identity and is calculated as follows: (Identical Residues x 100)/(Length of Alignment - Total Number of Gaps in Alignment).

Suitable cleaning cellulase glycosyl hydrolases are selected from the group consisting of: GH family 44 glycosyl hydrolases from *Paenibacillus polyxyma* (wild-type) such as XYG1006 described in WO 01/062903 or are variants thereof; GH family 12 glycosyl hydrolases from *Bacillus licheniformis* (wild-type) such as Seq. No. ID: 1 described in WO 99/02663 or are variants thereof; GH family 5 glycosyl hydrolases from *Bacillus agaradhaerens* (wild type) or variants thereof; GH family 5 glycosyl hydrolases from *Paenibacillus* (wild type) such as XYG1034 and XYG 1022described in WO 01/064853 or variants thereof; GH family 74 glycosyl hydrolases from *Jonesia sp.* (wild type) such as XYG1020 described in WO 2002/077242 or variants thereof; and GH family 74 glycosyl hydrolases from *Trichoderma Reesei* (wild type), such as the enzyme described in more detail in Sequence ID no. 2 of WO03/089598, or variants thereof.

Preferred glycosyl hydrolases are selected from the group consisting of: GH family 44 glycosyl hydrolases from *Paenibacillus polyxyma* (wild-type) such as XYG1006 or are variants thereof.

Typically, the cellulase modifies the fabric surface during the laundering process so as to improve the removal of soils adhered to the fabric after the laundering process during wearing and usage of the fabric, in subsequent wash cycles. Preferably, the cellulase modifies the fabric surface during the laundering process so as to improve the removal of soils adhered to the fabric after the laundering process during wearing and usage of the fabric, in the subsequent two, or even three wash cycles.

Typically, the cellulase is used at a concentration of 0.005ppm to 1.0ppm in the aqueous liquor during the first laundering process. Preferably, the cellulase is used at a concentration of 0.02ppm to 0.5ppm in the aqueous liquor during the first laundering process.

## Surfactant system

The cleaning composition may comprise a surfactant system. The cleaning composition may comprise from about 1% to about 80%, or from 1% to about 60%, preferably from about 5% to about 50% more preferably from about 8% to about 40%, by weight of the cleaning composition, of a surfactant system.

Surfactants suitable for use in the surfactant system may be derived from natural and/or renewable sources.

The surfactant system may comprise an anionic surfactant, more preferably an anionic surfactant selected from the group consisting of, alkyl benzene sulfonate, alkyl sulfate, alkyl alkoxy sulfate, especially alkyl ethoxy sulfate, paraffin sulfonate and mixtures thereof, alkyl benzene sulfonates are particularly preferred. The surfactant system may further comprise a surfactant selected from the group consisting of nonionic surfactant, cationic surfactant, amphoteric surfactant, zwitterionic surfactant, and mixtures thereof. The surfactant system preferably comprises a nonionic surfactant, for example an ethoxylated nonionic surfactant. The surfactant system may comprise an amphoteric surfactant, for example an amine oxide surfactant, such as an alkyl dimethyl amine oxide. The surfactant system may comprise a zwitterionic surfactant, such as a betaine.

The most preferred surfactant system for the detergent composition of the present invention comprises from 1% to 40%, preferably 6% to 35%, more preferably 8% to 30% weight of the total composition of an anionic surfactant, preferably comprising an alkyl benzene sulphonate. The preferred surfactant system may optionally in addition comprise an alkyl alkoxy sulfate surfactant, more preferably an alkyl ethoxy sulfate, optionally combined with 0.5% to 15%, preferably from 1% to 12%, more preferably from 2% to 10% by weight of the composition of amphoteric and/or zwitterionic surfactant, more preferably an amphoteric and even more preferably an amine oxide surfactant, especially an alkyl dimethyl amine oxide.

Preferably the composition further comprises a nonionic surfactant, especially an alcohol alkoxylate in particular an alcohol ethoxylate nonionic surfactant. Most preferably the surfactant system comprises an anionic and a nonionic surfactant, preferably the weight ratio of the anionic to nonionic surfactant is from 25:1 to 1:2.

## Anionic surfactant

Anionic surfactants may be in salt form or acid form, typically in the form of a water-soluble sodium, potassium, ammonium, magnesium or mono-, di- or tri- C2-C3 alkanolammonium salt, with the sodium cation being the usual one chosen.

Sulfonate Surfactant

Suitable anionic sulfonate surfactants for use herein include water-soluble salts of C8-C18 alkyl or hydroxyalkyl sulfonates; C11-C18 alkyl benzene sulfonates (LAS), modified alkylbenzene sulfonate (MLAS) as discussed in WO 99/05243, WO 99/05242, WO 99/05244, WO 99/05082, WO 99/05084, WO 99/05241, WO 99/07656, WO 00/23549, and WO 00/23548; methyl ester sulfonate (MES); and alpha-olefin sulfonate (AOS). Those also include the paraffin sulfonates may be monosulfonates and/or disulfonates, obtained by sulfonating paraffins of 10 to 20 carbon atoms. The sulfonate surfactant may also include the alkyl glyceryl sulfonate surfactants.

Sulfated anionic surfactant

Preferably the sulfated anionic surfactant is alkoxylated, more preferably, an alkoxylated branched sulfated anionic surfactant having an alkoxylation degree of from about 0.2 to about 4, even more preferably from about 0.3 to about 3, even more preferably from about 0.4 to about 1.5 and especially from about 0.4 to about 1. Preferably, the alkoxy group is ethoxy. When the sulfated anionic surfactant is a mixture of sulfated anionic surfactants, the alkoxylation degree is the weight average alkoxylation degree of all the components of the mixture (weight average alkoxylation degree). In the weight average alkoxylation degree calculation the weight of sulfated anionic surfactant components not having alkoxylated groups should also be included.

$$\text{Weight average alkoxylation degree} = (x1 * \text{alkoxylation degree of surfactant } 1 + x2 * \text{alkoxylation degree of surfactant } 2 + \ldots) / (x1 + x2 + \ldots)$$

wherein x1, x2, ... are the weights in grams of each sulfated anionic surfactant of the mixture and alkoxylation degree is the number of alkoxy groups in each sulfated anionic surfactant.

Preferably, the branching group is an alkyl. Typically, the alkyl is selected from methyl, ethyl, propyl, butyl, pentyl, cyclic alkyl groups and mixtures thereof. Single or multiple alkyl branches could be present on the main hydrocarbyl chain of the starting alcohol(s) used to produce the sulfated anionic surfactant used in the detergent of the invention. Most preferably the branched sulfated anionic surfactant is selected from alkyl sulfates, alkyl ethoxy sulfates, and mixtures thereof.

The branched sulfated anionic surfactant can be a single anionic surfactant or a mixture of anionic surfactants. In the case of a single surfactant the percentage of branching refers to the weight percentage of the hydrocarbyl chains that are branched in the original alcohol from which the surfactant is derived.

In the case of a surfactant mixture the percentage of branching is the weight average and it is defined according to the following formula:

$$\text{Weight average of branching (\%)} = [(x1 * \text{wt\% branched alcohol 1 in alcohol } 1 + x2 * \text{wt\% branched alcohol 2 in alcohol } 2 + \ldots) / (x1 + x2 + \ldots)] * 100$$

wherein x1, x2, ... are the weight in grams of each alcohol in the total alcohol mixture of the alcohols which were used as starting material for the anionic surfactant for the detergent of the invention. In the weight average branching degree calculation the weight of anionic surfactant components not having branched groups should also be included.

Suitable sulfate surfactants for use herein include water-soluble salts of C8-C18 alkyl or hydroxyalkyl, sulfate and/or ether sulfate. Suitable counterions include alkali metal cation or ammonium or substituted ammonium, but preferably sodium.

The sulfate surfactants may be selected from C8-C18 primary, branched chain and random alkyl sulfates (AS); C8-C18 secondary (2,3) alkyl sulfates; C8-C18 alkyl alkoxy sulfates (AExS) wherein preferably x is from 1-30 in which the alkoxy group could be selected from ethoxy, propoxy, butoxy or even higher alkoxy groups and mixtures thereof.

Alkyl sulfates and alkyl alkoxy sulfates are commercially available with a variety of chain lengths, ethoxylation and branching degrees. Commercially available sulfates include, those based on Neodol alcohols ex the Shell company, Lial - Isalchem and Safol ex the Sasol company, natural alcohols ex The Procter & Gamble Chemicals company.

Preferred alkyl sulfates are those in which the anionic surfactant is an alkyl ethoxy sulfate with a degree of ethoxylation of from about 0.2 to about 3, more preferably from about 0.3 to about 2, even more preferably from about 0.4 to about 1.5, and especially from about 0.4 to about 1. They are also preferred anionic surfactant having a level of branching of from about 5% to about 40%, even more preferably from about 10% to 35% and especially from about 20%

to 30%.

Nonionic surfactant

Preferably the surfactant system comprises a nonionic surfactant, in an amount of from 0.1% to 40%, preferably 0.2% to 20%, most preferably 0.5% to 10% by weight of the composition. Suitable nonionic surfactants include the condensation products of aliphatic alcohols with from 1 to 25 moles of ethylene oxide. The alkyl chain of the aliphatic alcohol can either be straight or branched, primary or secondary, and generally contains from 8 to 22 carbon atoms. Particularly preferred are the condensation products of alcohols having an alkyl group containing from 10 to 18 carbon atoms, preferably from 10 to 15 carbon atoms with from 2 to 18 moles, preferably 2 to 15, more preferably 5-12 of ethylene oxide per mole of alcohol. Highly preferred nonionic surfactants are the condensation products of guerbet alcohols with from 2 to 18 moles, preferably 2 to 15, more preferably 5-12 of ethylene oxide per mole of alcohol.

Other suitable non-ionic surfactants for use herein include fatty alcohol polyglycol ethers, alkylpolyglucosides and fatty acid glucamides.

Amphoteric surfactant

The surfactant system may include amphoteric surfactant, such as amine oxide. Preferred amine oxides are alkyl dimethyl amine oxide or alkyl amido propyl dimethyl amine oxide, more preferably alkyl dimethyl amine oxide and especially coco dimethyl amino oxide. Amine oxide may have a linear or mid-branched alkyl moiety. Typical linear amine oxides include water-soluble amine oxides containing one R1 C8-18 alkyl moiety and 2 R2 and R3 moieties selected from the group consisting of C1-3 alkyl groups and C1-3 hydroxyalkyl groups. Preferably amine oxide is characterized by the formula R1 - N(R2)(R3) O wherein R1 is a C8-18 alkyl and R2 and R3 are selected from the group consisting of methyl, ethyl, propyl, isopropyl, 2-hydroxethyl, 2-hydroxypropyl and 3-hydroxypropyl. The linear amine oxide surfactants in particular may include linear C10-C18 alkyl dimethyl amine oxides and linear C8-C12 alkoxy ethyl dihydroxy ethyl amine oxides. Preferred amine oxides include linear C10, linear C10-C12, and linear C12-C14 alkyl dimethyl amine oxides. As used herein "mid-branched" means that the amine oxide has one alkyl moiety having n1 carbon atoms with one alkyl branch on the alkyl moiety having n2 carbon atoms. The alkyl branch is located on the $\alpha$ carbon from the nitrogen on t he alkyl moiety. This type of branching for the amine oxide is also known in the art as an internal amine oxide. The total sum of n1 and n2 is from 10 to 24 carbon atoms, preferably from 12 to 20, and more preferably from 10 to 16. The number of carbon atoms for the one alkyl moiety (n1) should be approximately the same number of carbon atoms as the one alkyl branch (n2) such that the one alkyl moiety and the one alkyl branch are symmetric. As used herein "symmetric" means that $|n1 - n2|$ is less than or equal to 5, preferably 4, most preferably from 0 to 4 carbon atoms in at least 50 wt%, more preferably at least 75 wt% to 100 wt% of the mid-branched amine oxides for use herein.

The amine oxide may further comprise two moieties, independently selected from a C1-3 alkyl, a C1-3 hydroxyalkyl group, or a polyethylene oxide group containing an average of from about 1 to about 3 ethylene oxide groups. Preferably the two moieties are selected from a C1-3 alkyl, more preferably both are selected as a C1 alkyl.

Zwitterionic surfactant

Other suitable surfactants include betaines, such as alkyl betaines, alkylamidobetaine, amidazoliniumbetaine, sulfobetaine (INCI Sultaines) as well as the Phosphobetaine and preferably meets formula (I):

$$R^1\text{-}[CO\text{-}X(CH_2)_n]_x\text{-}N^+(R^2)(R_3)\text{-}(CH_2)_m\text{-}[CH(OH)\text{-}CH_2]_y\text{-}Y^- \qquad (I)$$

wherein

$R^1$ is a saturated or unsaturated C6-22 alkyl residue, preferably C8-18 alkyl residue, in particular a saturated C10-16 alkyl residue, for example a saturated C12-14 alkyl residue;
X is NH, $NR^4$ with C1-4 Alkyl residue $R^4$, O or S,
n a number from 1 to 10, preferably 2 to 5, in particular 3,
x 0 or 1, preferably 1,
$R^2$, $R^3$ are independently a C1-4 alkyl residue, potentially hydroxy substituted such as a hydroxyethyl, preferably a methyl.
m a number from 1 to 4, in particular 1, 2 or 3,
y 0 or 1 and
Y is COO, SO3, $OPO(OR^5)O$ or $P(O)(OR^5)O$, whereby $R^5$ is a hydrogen atom H or a C1-4 alkyl residue.

Preferred betaines are the alkyl betaines of the formula (Ia), the alkyl amido propyl betaine of the formula (Ib), the Sulfo betaines of the formula (Ic) and the Amido sulfobetaine of the formula (Id);

$R^1$-$N^+(CH_3)_2$-$CH_2COO^-$ (Ia)

$R^1$-CO-$NH(CH_2)_3$-$N^+(CH_3)_2$-$CH_2COO^-$ (Ib)

$R^1$-$N^+(CH_3)_2$-$CH_2CH(OH)CH_2SO_3$- (Ic)

$R^1$-CO-NH-$(CH_2)_3$-$N^+(CH_3)_2$-$CH_2CH(OH)CH_2SO_3$- (Id)

in which $R^1$1 as the same meaning as in formula I. Particularly preferred betaines are the Carbobetaine [wherein $Y^-$ =$COO^-$], in particular the Carbobetaine of the formula (Ia) and (Ib), more preferred are the Alkylamidobetaine of the formula (Ib).

Examples of suitable betaines and sulfobetaine are the following [designated in accordance with INCI]: Almondamidopropyl of betaines, Apricotam idopropyl betaines, Avocadamidopropyl of betaines, Babassuamidopropyl of betaines, Behenam idopropyl betaines, Behenyl of betaines, betaines, Canolam idopropyl betaines, Capryl/Capram idopropyl betaines, Carnitine, Cetyl of betaines, Cocamidoethyl of betaines, Cocam idopropyl betaines, Cocam idopropyl Hydroxysultaine, Coco betaines, Coco Hydroxysultaine, Coco/Oleam idopropyl betaines, Coco Sultaine, Decyl of betaines, Dihydroxyethyl Oleyl Glycinate, Dihydroxyethyl Soy Glycinate, Dihydroxyethyl Stearyl Glycinate, Dihydroxyethyl Tallow Glycinate, Dimethicone Propyl of PG-betaines, Erucam idopropyl Hydroxysultaine, Hydrogenated Tallow of betaines, Isostearam idopropyl betaines, Lauram idopropyl betaines, Lauryl of betaines, Lauryl Hydroxysultaine, Lauryl Sultaine, Milkam idopropyl betaines, Minkamidopropyl of betaines, Myristam idopropyl betaines, Myristyl of betaines, Oleam idopropyl betaines, Oleam idopropyl Hydroxysultaine, Oleyl of betaines, Olivamidopropyl of betaines, Palmam idopropyl betaines, Palm itam idopropyl betaines, Palmitoyl Carnitine, Palm Kernelam idopropyl betaines, Polytetrafluoroethylene Acetoxypropyl of betaines, Ricinoleam idopropyl betaines, Sesam idopropyl betaines, Soyam idopropyl betaines, Stearam idopropyl betaines, Stearyl of betaines, Tallowam idopropyl betaines, Tallowam idopropyl Hydroxysultaine, Tallow of betaines, Tallow Dihydroxyethyl of betaines, Undecylenam idopropyl betaines and Wheat Germam idopropyl betaines. A preferred betaine is, for example, Cocoamidopropylbetaine.

## Fatty Acid

Especially when in liquid form, preferably, the detergent composition comprises between 1.5% and 20%, more preferably between 2% and 15%, even more preferably between 3% and 10%, most preferably between 4% and 8% by weight of the liquid detergent composition of soap, preferably a fatty acid salt, more preferably an amine neutralized fatty acid salt, wherein preferably the amine is an alkanolamine more preferably selected from monoethanolamine, diethanolamine, triethanolamine or a mixture thereof, more preferably monoethanolamine.

## Perfume

Preferred compositions of the invention comprise perfume. Typically the composition comprises a perfume that comprises one or more perfume raw materials, selected from the group as described in WO08/87497. However, any perfume useful in a detergent may be used. A preferred method of incorporating perfume into the compositions of the invention is via an encapsulated perfume particle comprising either a water-soluble hydroxylic compound or melamine-formaldehyde or modified polyvinyl alcohol. In one aspect the encapsulate comprises (a) an at least partially water-soluble solid matrix comprising one or more water-soluble hydroxylic compounds, preferably starch; and (b) a perfume oil encapsulated by the solid matrix. In a further aspect the perfume may be pre-complexed with a polyamine, preferably a polyethylenimine so as to form a Schiff base.

## Polymers

The detergent composition may comprise one or more polymers for example for cleaning and/or care.Examples are optionally modified carboxymethylcellulose, poly (ethylene glycol), poly(vinyl alcohol), polycarboxylates such as polyacrylates, maleic/acrylic acid copolymers and lauryl methacrylate/acrylic acid co-polymers and carboxylate polymers.

Suitable carboxylate polymers include maleate/acrylate random copolymer or polyacrylate homopolymer. The carboxylate polymer may be a polyacrylate homopolymer having a molecular weight of from 4,000 Da to 9,000 Da, or from 6,000 Da to 9,000 Da. Other suitable carboxylate polymers are co-polymers of maleic acid and acrylic acid, and may have a molecular weight in the range of from 4,000 Da to 90,000 Da.

Other suitable carboxylate polymers are co-polymers comprising: (i) from 50 to less than 98 wt% structural units derived from one or more monomers comprising carboxyl groups; (ii) from 1 to less than 49 wt% structural units derived from one or more monomers comprising sulfonate moieties; and (iii) from 1 to 49 wt% structural units derived from one or more types of monomers selected from ether bond-containing monomers represented by formulas (I) and (II):

formula (I):

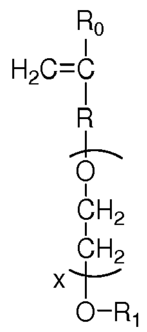

wherein in formula (I), $R_0$ represents a hydrogen atom or $CH_3$ group, R represents a $CH_2$ group, $CH_2CH_2$ group or single bond, X represents a number 0-5 provided X represents a number 1-5 when R is a single bond, and $R_1$ is a hydrogen atom or C1 to C20 organic group;

formula (II)

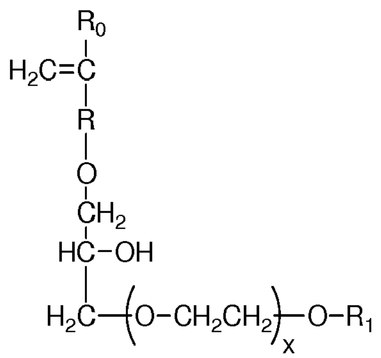

in formula (II), $R_0$ represents a hydrogen atom or $CH_3$ group, R represents a $CH_2$ group, $CH_2CH_2$ group or single bond, X represents a number 0-5, and $R_1$ is a hydrogen atom or C1 to C20 organic group.

The composition may comprise one or more amphiphilic cleaning polymers such as the compound having the following general structure: bis($(C_2H_5O)(C_2H_4O)n$)($CH_3$)-$N^+$-$C_xH_{2x}$-$N^+$-($CH_3$)-bis($(C_2H_5O)(C_2H_4O)n$), wherein n = from 20 to 30, and x = from 3 to 8, or sulphated or sulphonated variants thereof. In one aspect, this polymer is sulphated or sulphonated to provide a zwitterionic soil suspension polymer.

The composition preferably comprises amphiphilic alkoxylated grease cleaning polymers which have balanced hydrophilic and properties such that they remove grease particles from fabrics and surfaces. Preferred amphiphilic alkoxylated grease cleaning polymers comprise a core structure and a plurality of alkoxylate groups attached to that core structure. These may comprise alkoxylated polyalkylenimines, preferably having an inner polyethylene oxide block and an outer polypropylene oxide block. Typically these may be incorporated into the compositions of the invention in amounts of from 0.005 to 10 wt%, generally from 0.5 to 8 wt%.

Alkoxylated polycarboxylates such as those prepared from polyacrylates are useful herein to provide additional grease removal performance. Such materials are described in WO 91/08281 and PCT 90/01815. Chemically, these materials comprise polyacrylates having one ethoxy side-chain per every 7-8 acrylate units. The side-chains are of the formula $-(CH_2CH_2O)_m (CH_2)_nCH_3$ wherein m is 2-3 and n is 6-12. The side-chains are ester-linked to the polyacrylate "backbone" to provide a "comb" polymer type structure. The molecular weight can vary, but is typically in the range of about 2000 to about 50,000. Such alkoxylated polycarboxylates can comprise from about 0.05% to about 10%, by weight, of the compositions herein.

The composition may comprise polyethylene glycol polymers and these may be particularly preferred in compositions comprising mixed surfactant systems. Suitable polyethylene glycol polymers include random graft co-polymers comprising: (i) hydrophilic backbone comprising polyethylene glycol; and (ii) side chain(s) selected from the group consisting of: C4-C25 alkyl group, polypropylene, polybutylene, vinyl ester of a saturated C1-C6 mono-carboxylic acid, C1-C6 alkyl ester of acrylic or methacrylic acid, and mixtures thereof. Suitable polyethylene glycol polymers have a polyethylene glycol backbone with random grafted polyvinyl acetate side chains. The average molecular weight of the polyethylene glycol backbone can be in the range of from 2,000 Da to 20,000 Da, or from 4,000 Da to 8,000 Da. The molecular weight ratio of the polyethylene glycol backbone to the polyvinyl acetate side chains can be in the range of from 1:1 to 1:5, or from 1:1.2 to 1:2. The average number of graft sites per ethylene oxide units can be less than 1, or less than 0.8, the average number of graft sites per ethylene oxide units can be in the range of from 0.5 to 0.9, or the average number of graft sites per ethylene oxide units can be in the range of from 0.1 to 0.5, or from 0.2 to 0.4. A suitable polyethylene glycol polymer is Sokalan HP22.

Typically these polymers when present are each incorporated into the compositions of the invention in amounts from 0.005 to 10 wt%, more usually from 0.05 to 8 wt%.

Preferably the composition comprises one or more carboxylate polymer, such as a maleate/acrylate random copolymer or polyacrylate homopolymer. In one aspect, the carboxylate polymer is a polyacrylate homopolymer having a molecular weight of from 4,000 Da to 9,000 Da, or from 6,000 Da to 9,000 Da. Typically these are incorporated into the compositions of the invention in amounts from 0.005 to 10 wt%, or from 0.05 to 8 wt%.

Preferably the composition comprises one or more soil release polymers.

Suitable soil release polymers are polyester soil release polymers such as Repel-o-tex polymers, including Repel-o-tex SF, SF-2 and SRP6 supplied by Rhodia. Other suitable soil release polymers include Texcare polymers, including Texcare SRA100, SRA300, SRN100, SRN170, SRN240, SRN260, SRN300 and SRN325 supplied by Clariant. Other suitable soil release polymers are Marloquest polymers, such as Marloquest SL supplied by Sasol.

Preferably the composition comprises one or more cellulosic polymer, including those selected from alkyl cellulose, alkyl alkoxyalkyl cellulose, carboxyalkyl cellulose, alkyl carboxyalkyl cellulose. Preferred cellulosic polymers are selected from the group comprising carboxymethyl cellulose, methyl cellulose, methyl hydroxyethyl cellulose, methyl carboxymethyl cellulose, and mixures thereof. In one aspect, the carboxymethyl cellulose has a degree of carboxymethyl substitution from 0.5 to 0.9 and a molecular weight from 100,000 Da to 300,000 Da.

The composition preferably comprises a cationically-modified polysaccharide polymer. Preferably, the cationic polysaccharide polymer is selected from cationically modified hydroxyethyl cellulose, cationically modified hydroxypropyl cellulose, cationically and hydrophobically modified hydroxyethyl cellulose, cationically and hydrophobically modified hydroxypropyl cellulose, or a mixture thereof, more preferably cationically modified hydroxyethyl cellulose, cationically and hydrophobically modified hydroxyethyl cellulose, or a mixture thereof.

Amines

The cleaning compositions described herein may contain an amine. The cleaning compositions may include from about 0.1% to about 10%, or from about 0.2% to about 5%, or from about 0.5% to about 4%, or from about 0.1 % to about 4%, or from about 0.1 % to about 2%, by weight of the composition, of an amine. The amine can be subjected to protonation depending on the pH of the cleaning medium in which it is used. Non-limiting examples of amines include, but are not limited to, etheramines, cyclic amines, polyamines, oligoamines (e.g., triamines, diamines, pentamines, tetraamines), or combinations thereof. The compositions described herein may comprise an amine selected from the group consisting of oligoamines, etheramines, cyclic amines, and combinations thereof. In some aspects, the amine is not an alkanolamine. In some aspects, the amine is not a polyalkyleneimine. Examples of suitable oligoamines include tetraethylenepentamine, triethylenetetraamine, diethylenetriamine, and mixtures thereof. Etheramines and cyclic amines may be particularly preferred.

Fabric Shading Dye

The composition may comprise a fabric shading agent. Suitable fabric shading agents include dyes, dye-clay conjugates, and pigments. Suitable dyes include small molecule dyes and polymeric dyes. Suitable small molecule dyes include small molecule dyes selected from the group consisting of dyes falling into the Colour Index (C.I.) classifications of Direct Blue, Direct Red, Direct Violet, Acid Blue, Acid Red, Acid Violet, Basic Blue, Basic Violet and Basic Red, or mixtures thereof. Preferered dyes include alkoxylated azothiophenes, Solvent Violet 13, Acid Violet 50 and Direct Violet 9. Particularly preferred dyes are polymeric dyes, particularly comprising polyalkoxy, most preferably polyethoxy groups, for example:

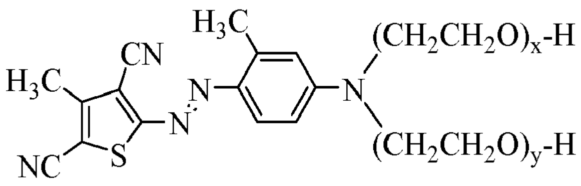

wherein the index values x and y are independently selected from 1 to 10.

Dye Transfer Inhibitors

Suitable dye transfer inhibitors include polyamine N-oxide polymers, copolymers of N-vinylpyrrolidone and N-vinylimidazole, polyvinylpyrrolidone, polyvinyloxazolidone, polyvinylimidazole and mixtures thereof. Preferred are poly(vinyl pyrrolidone), poly(vinylpyridine betaine), poly(vinylpyridine N-oxide), poly(vinyl pyrrolidone-vinyl imidazole) and mixtures thereof. Suitable commercially available dye transfer inhibitors include PVP-K15 and K30 (Ashland), Sokalan® HP165, HP50, HP53, HP59, HP56K, HP56, HP66 (BASF), Chromabond® S-400, S403E and S-100 (Ashland).

Chelant

The composition may comprise chelant for example selected from phosphonic, sulphonic, succinic and acetic chelants or mixtures thereof. Suitable examples include HEDP, DTPA, EDTA, MGDA, GLDA, EDDS and 4,5-dihydroxy-1,3-benzenedisulfonic acids and salts thereof.

Methods of Making the Composition

The present invention relates to methods of making the compositions described herein. The compositions of the invention may be solid (for example granules or tablets) or liquid form. Preferably the compositions are in liquid form. They may be made by any process chosen by the formulator, including by a batch process, a continuous loop process, or combinations thereof.

When in the form of a liquid, the compositions of the invention may be aqueous (typically above 2 wt% or even above 5 or 10 wt% total water, up to 90 or up to 80wt% or 70 wt% total water) or non-aqueous (typically below 2 wt% total water content). Typically the compositions of the invention will be in the form of an aqueous solution or uniform dispersion or suspension of optical brightener, DTI and optional additional adjunct materials, some of which may normally be in solid form, that have been combined with the normally liquid components of the composition, such as the liquid alcohol ethoxylate nonionic, the aqueous liquid carrier, and any other normally liquid optional ingredients. Such a solution, dispersion or suspension will be acceptably phase stable. When in the form of a liquid, the detergents of the invention preferably have viscosity from 1 to 1500 centipoises (1-1500 mPa*s), more preferably from 100 to 1000 centipoises (100-1000 mPa*s), and most preferably from 200 to 500 centipoises (200-500 mPa*s) at 20s-1 and 21°C. Viscosity can be determined by conventional methods. Viscosity may be measured using an AR 550 rheometer from TA instruments using a plate steel spindle at 40 mm diameter and a gap size of 500 $\mu$m. The high shear viscosity at 20s-1 and low shear viscosity at 0.05-1 can be obtained from a logarithmic shear rate sweep from 0.1-1 to 25-1 in 3 minutes time at 21C. The preferred rheology described therein may be achieved using internal existing structuring with detergent ingredients or by employing an external rheology modifier. More preferably the detergents, such as detergent liquid compositions have a high shear rate viscosity of from about 100 centipoise to 1500 centipoise, more preferably from 100 to 1000 cps. Unit Dose detergents, such as detergent liquid compositions have high shear rate viscosity of from 400 to 1000cps. Detergents such as laundry softening compositions typically have high shear rate viscosity of from 10 to 1000, more preferably from 10 to 800 cps, most preferably from 10 to 500 cps. Hand dishwashing compositions have high shear rate viscosity of from 300 to 4000 cps, more preferably 300 to 1000 cps.

The cleaning and/or treatment compositions in the form of a liquid herein can be prepared by combining the components thereof in any convenient order and by mixing, e.g., agitating, the resulting component combination to form a phase stable liquid detergent composition. In a process for preparing such compositions, a liquid matrix is formed containing at least a major proportion, or even substantially all, of the liquid components, e.g., nonionic surfactant, the non-surface active liquid carriers and other optional liquid components, with the liquid components being thoroughly admixed by imparting shear agitation to this liquid combination. For example, rapid stirring with a mechanical stirrer may usefully be employed. While shear agitation is maintained, substantially all of any anionic surfactants and the solid form ingredients can be added. Agitation of the mixture is continued, and if necessary, can be increased at this point to form a solution or a uniform dispersion of insoluble solid phase particulates within the liquid phase. After some or all of the solid-form materials have been added to this agitated mixture, particles of any enzyme material to be included, e.g.,

enzyme granulates, are incorporated. As a variation of the composition preparation procedure hereinbefore described, one or more of the solid components may be added to the agitated mixture as a solution or slurry of particles premixed with a minor portion of one or more of the liquid components. After addition of all of the composition components, agitation of the mixture is continued for a period of time sufficient to form compositions having the requisite viscosity and phase stability characteristics. Frequently this will involve agitation for a period of from about 30 to 60 minutes.

The adjunct ingredients in the compositions of this invention may be incorporated into the composition as the product of the synthesis generating such components, either with or without an intermediate purification step. Where there is no purification step, commonly the mixture used will comprise the desired component or mixtures thereof (and percentages given herein relate to the weight percent of the component itself unless otherwise specified) and in addition unreacted starting materials and impurities formed from side reactions and/or incomplete reaction. For example, for an ethoxylated or substituted component, the mixture will likely comprise different degrees of ethoxylation/substitution.

Method of Use

The present invention relates to methods of using the cleaning compositions of the present invention to clean a surface, such as a textile. In general, the method includes mixing the cleaning composition as described herein with water to form an aqueous liquor and contacting a surface, preferably a textile, with the aqueous liquor in a laundering step. The target surface may include a greasy soil such as a body soil. The compositions herein, typically prepared as hereinbefore described, can be used to form aqueous washing/treatment solutions for use in the laundering/treatment of fabrics and/or hard surfaces. Generally, an effective amount of such a composition is added to water, for example in a conventional fabric automatic washing machine, to form such aqueous liquor laundering solutions. The aqueous liquor so formed is then contacted, typically under agitation, with the fabrics to be laundered/treated therewith. An effective amount of the cleaning composition herein added to water to form aqueous liquors for washing can comprise amounts sufficient to form from about 500 to 25,000 ppm, or from 500 to 15,000 ppm of composition in aqueousliquor, or from about 1,000 to 3,000 ppm of the cleaning compositions herein will be provided in aqueous liquor.

Typically, the aqueous liquor is formed by contacting the detergent with wash water in such an amount so that the concentration of the cleaning composition in the aqueous liquor is from above 0.1 g/l to 5g/l, or from 1g/l, and to 4.5g/l, or to 4.0g/l, or to 3.5g/l, or to 3.0g/l, or to 2.5g/l, or even to 2.0g/l, or even to 1.5g/l. The method of laundering fabric or textile may be carried out in a top-loading or front-loading automatic washing machine, or can be used in a hand-wash laundry application. In these applications, the aqueous liquor formed and concentration of laundry detergent composition in the aqueous liquor is that of the main wash cycle. Any input of water during any optional rinsing step(s) is not included when determining the volume of the aqueous liquor.

The aqueous liquor may comprise 40 litres or less of water, or 30 litres or less, or 20 litres or less, or 10 litres or less, or 8 litres or less, or even 6 litres or less of water. The wash liquor may comprise from above 0 to 15 litres, or from 2 litres, and to 12 litres, or even to 8 litres of water. Typically from 0.01kg to 2kg of fabric per litre of aqueous liquor is dosed into said aqueous liquor. Typically from 0.01kg, or from 0.05kg, or from 0.07kg, or from 0.10kg, or from 0.15kg, or from 0.20kg, or from 0.25kg fabric per litre of aqueous liquor is dosed into said aqueous liquor. Optionally, 50g or less, or 45g or less, or 40g or less, or 35g or less, or 30g or less, or 25g or less, or 20g or less, or even 15g or less, or even 10g or less of the composition is contacted to water to form the aqueous liquor. Such compositions are typically employed at concentrations of from about 500 ppm to about 15,000 ppm in solution. When the wash solvent is water, the water temperature typically ranges from about 5 °C to about 90 °C and, when the situs comprises a fabric, the water to fabric ratio is typically from about 1:1 to about 30:1. Typically the aqueous liquor comprising the detergent of the invention has a pH of from 3 to 11.5.

In one aspect, such method comprises the steps of optionally washing and/or rinsing said surface or fabric, contacting said surface or fabric with any composition disclosed in this specification then optionally washing and/or rinsing said surface or fabric is disclosed, with an optional drying step.

Drying of such surfaces or fabrics may be accomplished by any one of the common means employed either in domestic or industrial settings: machine drying or open-air drying. The fabric may comprise any fabric capable of being laundered in normal consumer or institutional use conditions, and the invention is particularly suitable for synthetic textiles such as polyester and nylon and especially for treatment of mixed fabrics and/or fibres comprising synthetic and cellulosic fabrics and/or fibres. As examples of synthetic fabrics are polyester, nylon, these may be present in mixtures with cellulosic fibres, for example, polycotton fabrics. The solution typically has a pH of from 7 to 11, more usually 8 to 10.5. The compositions are typically employed at concentrations from 500 ppm to 5,000 ppm in solution. The water temperatures typically range from about 5 °C to about 90 °C. The water to fabric ratio is typically from about 1:1 to about 30:1.

Use of an Acyl Hydrazone Bleach Catalyst

The present invention further relates to a use of an acyl hydrazone bleach catalyst as described herein, for example in a cleaning composition, to enhance the stain removal, bleaching, and/or malodor-reducing benefits of an endo-beta-1,6-galactanase enzyme, such as a galactanase enzyme as described herein.

TEST METHODS

Enzymatic activity towards xyloglucan substrates

An enzyme is deemed to have activity towards xyloglucan if the pure enzyme has a specific activity of greater than 50000 XyloU/g according to the following assay at pH 7.5.

The xyloglucanase activity is measured using AZCL-xyloglucan from Megazyme, Ireland as substrate (blue substrate).

A solution of 0.2% of the blue substrate is suspended in a 0.1M phosphate buffer pH 7.5, 20°C under stirring in a 1.5ml Eppendorf tubes (0.75ml to each), 50 microlitres enzyme solution is added and they are incubated in an Eppendorf Thermomixer for 20 minutes at 40°C, with a mixing of 1200 rpm. After incubation the coloured solution is separated from the solid by 4 minutes centrifugation at 14,000 rpm and the absorbance of the supernatant is measured at 600nm in a 1cm cuvette using a spectrophotometer. One XyloU unit is defined as the amount of enzyme resulting in an absorbance of 0.24 in a 1cm cuvette at 600nm.

Only absorbance values between 0.1 and 0.8 are used to calculate the XyloU activity. If an absorbance value is measured outside this range, optimization of the starting enzyme concentration should be carried out accordingly.

Enzymatic activity towards amorphous cellulose substrates

An enzyme is deemed to have activity towards amorphous cellulose if the pure enzyme has a specific activity of greater than 20000 EBG/g according to the following assay at pH 7.5. Chemicals used as buffers and substrates were commercial products of at least reagent grade. Endoglucanase Activity Assay Materials:

- 0.1M phosphate buffer pH 7.5
- Cellazyme C tablets, supplied by Megazyme International, Ireland.
- Glass microfiber filters, GF/C, 9cm diameter, supplied by Whatman.

Method:

In test tubes, mix 1ml pH 7.5 buffer and 5ml deionised water.

Add 100 microliter of the enzyme sample (or of dilutions of the enzyme sample with known weight:weight dilution factor). Add 1 Cellazyme C tablet into each tube, cap the tubes and mix on a vortex mixer for 10 seconds. Place the tubes in a thermostated water bath, temperature 40°C. After 15, 30 and 45 minutes, mix the contents of the tubes by inverting the tubes, and replace in the water bath. After 60 minutes, mix the contents of the tubes by inversion and then filter through a GF/C filter. Collect the filtrate in a clean tube.

Measure Absorbance (Aenz) at 590nm, with a spectrophotometer. A blank value, Awater, is determined by adding 100μl water instead of 100 microliter enzyme dilution.

$$\text{Calculate Adelta} = \text{Aenz} - \text{Awater}.$$

Adelta must be <0.5. If higher results are obtained, repeat with a different enzyme dilution factor.

Determine DFO.1, where DFO.1 is the dilution factor needed to give Adelta = 0.1 . Unit Definition: 1 Endo-Beta-Glucanase activity unit (1 EBG) is the amount of enzyme that gives Adelta = 0.10, under the assay conditions specified above. Thus, for example, if a given enzyme sample, after dilution by a dilution factor of 100, gives Adelta= 0.10, then the enzyme sample has an activity of 100 EBG/g.

EXAMPLES

The following are illustrative examples of cleaning compositions of the invention and are not intended to be limiting.

Examples 1 to 18: Unit Dose Compositions.

These examples provide various formulations for unit dose laundry detergents and comprise double compartment unit dose products comprising one powder and one liquid compartment. The film used to encapsulate the compositions in PVA. Each example is prepared by combining a liquid compartment composition selected from compositions A-E with a powder compartment composition selected from compositions F-K.

| Example | 1 | 2 | 3 | 4 | 5 | 6 |
|---|---|---|---|---|---|---|
| Liquid composition | 20g A | 25g A | 20g A | 15g A | 20g B | 20g B |
| Solid composition | 15g F | 12g G | 12g H | 12g I | 15g J | 15g K |

| Example | 7 | 8 | 9 | 10 | 11 | 12 |
|---|---|---|---|---|---|---|
| Liquid composition | 15g B | 17g B | 20g C | 19g C | 15g C | 25g C |
| Solid composition | 15g L | 14g F | 15g G | 18g H | 15g I | 12g J |

| Example | 13 | 14 | 15 | 16 | 17 | 18 |
|---|---|---|---|---|---|---|
| Liquid composition | 20g D | 18g D | 22g D | 32g E | 32g E | 27g E |
| Solid composition | 20g K | 13g L | 15g F | 17g G | 12g H | 18g I |

| Ingredients | A | B | C | D | E |
|---|---|---|---|---|---|
| | % weight of compartment | | | | |
| LAS | 19.09 | 16.76 | 8.59 | 6.56 | 3.44 |
| AE3S | 1.91 | 0.74 | 0.18 | 0.46 | 0.07 |
| AE7 | 14.00 | 17.50 | 26.33 | 28.08 | 31.59 |
| Citric Acid | 0.6 | 0.6 | 0.6 | 0.6 | 0.6 |
| C12-15 Fatty Acid | 14.8 | 14.8 | 14.8 | 14.8 | 14.8 |
| Polymer 3 | 4.0 | 4.0 | 4.0 | 4.0 | 4.0 |
| Chelant 2 | 1.2 | 1.2 | 1.2 | 1.2 | 1.2 |
| Optical Brightener 1 | 0.20 | 0.25 | 0.01 | 0.01 | 0.50 |
| Optical Brightener 2 | 0.20 | - | 0.25 | 0.03 | 0.01 |
| Optical Brightener 3 | 0.18 | 0.09 | 0.30 | 0.01 | - |
| DTI 1 | 0.10 | - | 0.20 | 0.01 | 0.05 |
| DTI 2 | - | 0.10 | 0.20 | 0.25 | 0.05 |
| Glycerol | 6.1 | 6.1 | 6.1 | 6.1 | 6.1 |
| Monoethanol amine | 8.0 | 8.0 | 8.0 | 8.0 | 8.0 |
| Tri-isopropanol amine | - | - | 2.0 | - | - |
| Tri-ethanol amine | - | 2.0 | - | - | - |
| Cumene sulfonate | - | - | - | - | 2.0 |
| Protease | 0.80 | 0.60 | 0.07 | 1.00 | 1.50 |

| | | | | | |
|---|---|---|---|---|---|
| Mannanase | - | - | 0.05 | 0.10 | 0.01 |
| Amylase 1 | 0.20 | 0.11 | 0.30 | 0.50 | 0.05 |
| Amylase 2 | 0.11 | 0.20 | 0.10 | - | 0.50 |
| Polishing enzyme | 0.005 | 0.05 | - | - | - |
| Galactanase | 0.005 | 0.05 | 0.005 | 0.010 | 0.005 |
| Dispersin B | 0.010 | - | 0.005 | 0.005 | - |
| Cyclohexyl dimethanol | - | - | - | 2.0 | - |
| Acid violet 50 | 0.03 | 0.02 | | | |
| Violet DD | | | 0.01 | 0.05 | 0.02 |
| Structurant | 0.14 | 0.14 | 0.14 | 0.14 | 0.14 |
| Perfume | 1.9 | 1.9 | 1.9 | 1.9 | 1.9 |
| Water, solvents and miscellaneous | To 100% | | | | |
| pH | 7.5-8.2 | | | | |

| Ingredient | F | G | H | I | J | K |
|---|---|---|---|---|---|---|
| | % weight | | | | | |
| Sodium carbonate | 20.0 | 35.0 | 30.0 | 29.0 | 28.0 | 18.0 |
| Carboxymethyl cellulose | 2.0 | 1.0 | - | - | 2.5 | 0.6 |
| Sodium silicate 2R | 5.0 | - | 5.0 | 3.2 | 20.0 | - |
| Tetraacetyl ethylenediamine | 20.0 | 15.0 | 18.0 | 15.0 | - | 25.0 |
| Sodium percarbonate | 50.0 | 44.0 | 45.0 | 45.0 | 29.0 | 50.0 |
| Acyl hydrazone | 0.6 | 0.2 | 0.1 | 0.1 | 0.05 | 0.6 |
| Sulfate/ Water & Miscellaneous | Balance | | | | | |

Based on total cleaning and/or treatment composition/compartment weight. Enzyme levels are reported as raw material.

Examples 19 to 24:

Granular laundry detergent compositions for hand washing or washing machines, typically top-loading washing machines.

| Ingredient | 19 | 20 | 21 | 22 | 23 | 24 |
|---|---|---|---|---|---|---|
| | % weight | | | | | |
| LAS | 11.33 | 10.81 | 7.04 | 4.20 | 3.92 | 2.29 |
| Quaternary ammonium | 0.70 | 0.20 | 1.00 | 0.60 | - | - |
| AE3S | 0.51 | 0.49 | 0.32 | - | 0.08 | 0.10 |
| AE7 | 8.36 | 11.50 | 12.54 | 11.20 | 16.00 | 21.51 |
| Sodium Tripolyphosphate | 5.0 | - | 4.0 | 9.0 | 2.0 | - |
| Zeolite A | - | 1.0 | - | 1.0 | 4.0 | 1.0 |
| Sodium silicate 1.6R | 7.0 | 5.0 | 2.0 | 3.0 | 3.0 | 5.0 |
| Sodium carbonate | 20.0 | 17.0 | 23.0 | 14.0 | 14.0 | 16.0 |
| Polyacrylate MW 4500 | 1.0 | 0.6 | 1.0 | 1.0 | 1.5 | 1.0 |
| Polymer 6 | 0.1 | 0.2 | - | - | 0.1 | - |
| Carboxymethyl cellulose | 1.0 | 0.3 | 1.0 | 1.0 | 1.0 | 1.0 |
| Acid Violet 50 | 0.05 | - | 0.02 | - | 0.04 | - |
| Violet DD | - | 0.03 | - | 0.03 | - | 0.03 |
| Protease 2 | 0.10 | 0.10 | 0.10 | 0.10 | - | 0.10 |
| Amylase | 0.03 | - | 0.03 | 0.03 | 0.03 | 0.03 |
| Lipase | 0.03 | 0.07 | 0.30 | 0.10 | 0.07 | 0.40 |
| Polishing enzyme | 0.002 | - | 0.05 | - | 0.02 | - |
| Galactanase | 0.001 | 0.001 | 0.01 | 0.05 | 0.002 | 0.02 |
| Dispersin B | 0.001 | 0.001 | 0.05 | - | 0.001 | - |
| Optical Brightener 1 | 0.200 | 0.001 | 0.300 | 0.650 | 0.050 | 0.001 |
| Optical Brightener 2 | 0.060 | - | 0.650 | 0.180 | 0.200 | 0.060 |
| Optical Brightener 3 | 0.100 | 0.060 | 0.050 | - | 0.030 | 0.300 |
| Chelant 1 | 0.60 | 0.80 | 0.60 | 0.25 | 0.60 | 0.60 |
| DTI 1 | 0.32 | 0.15 | 0.15 | - | 0.10 | 0.10 |
| DTI 2 | 0.32 | 0.15 | 0.30 | 0.30 | 0.10 | 0.20 |
| Sodium Percarbonate | 4.6 | 5.2 | 5.0 | 5.7 | 4.5 | 7.3 |
| Nonanoyloxybenzensulfonate | 1.9 | 0.0 | 1.66 | 0.0 | 0.33 | 0.75 |
| Tetraacetylethylenediamine | 0.58 | 1.2 | 0.51 | 0.0 | 0.015 | 0.28 |
| Photobleach | 0.0030 | 0.0 | 0.0012 | 0.0030 | 0.0021 | - |
| S-ACMC | 0.1 | 0.0 | 0.0 | 0.0 | 0.06 | 0.0 |
| Acyl hydrazone | 0.6 | 0.7 | 0.9 | 0.9 | 0.3 | 0.6 |
| Sulfate/Moisture | Balance | | | | | |

Examples 25-30

Granular laundry detergent compositions typically for front-loading automatic washing machines.

| Ingredient | 25 | 26 | 27 | 28 | 29 | 30 |
|---|---|---|---|---|---|---|
| | % weight | | | | | |
| LAS | 6.08 | 5.05 | 4.27 | 3.24 | 2.30 | 1.09 |
| AE3S | - | 0.90 | 0.21 | 0.18 | - | 0.06 |
| AS | 0.34 | - | - | - | - | - |
| AE7 | 4.28 | 5.95 | 6.72 | 7.98 | 9.20 | 10.35 |
| Quaternary ammonium | 0.5 | - | - | 0.3 | - | - |
| Crystalline layered silicate | 4.1 | - | 4.8 | - | - | - |
| Zeolite A | 5.0 | - | 2.0 | - | 2.0 | 2.0 |
| Citric acid | 3.0 | 4.0 | 3.0 | 4.0 | 2.5 | 3.0 |
| Sodium carbonate | 11.0 | 17.0 | 12.0 | 15.0 | 18.0 | 18.0 |
| Sodium silicate 2R | 0.08 | - | 0.11 | - | - | - |
| Optical Brightener 1 | - | 0.25 | 0.05 | 0.01 | 0.10 | 0.02 |
| Optical Brightener 2 | - | - | 0.25 | 0.20 | 0.01 | 0.08 |
| Optical Brightener 3 | - | 0.06 | 0.04 | 0.15 | - | 0.05 |
| DTI 1 | 0.08 | - | 0.04 | - | 0.10 | 0.01 |
| DTI 2 | 0.08 | - | 0.04 | 0.10 | 0.10 | 0.02 |
| Soil release agent | 0.75 | 0.72 | 0.71 | 0.72 | - | - |
| Acrylic /maleic acid copolymer | 1.1 | 3.7 | 1.0 | 3.7 | 2.6 | 3.8 |
| Carboxymethyl cellulose | 0.2 | 1.4 | 0.2 | 1.4 | 1.0 | 0.5 |
| Protease 3 | 0.20 | 0.20 | 0.30 | 0.15 | 0.12 | 0.13 |
| Amylase 3 | 0.20 | 0.15 | 0.20 | 0.30 | 0.15 | 0.15 |
| Lipase | 0.05 | 0.15 | 0.10 | | - | - |
| Amylase 2 | 0.03 | 0.07 | - | - | 0.05 | 0.05 |
| Cellulase 2 | - | - | - | - | 0.10 | 0.10 |
| Polishing enzyme | 0.003 | 0.005 | 0.020 | - | - | - |
| Galactanase (SEQ ID NO: 1, 2, 3 or mixtures) | 0.002 | 0.010 | 0.020 | 0.020 | 0.010 | 0.003 |
| Dispersin B | 0.002 | 0.010 | - | 0.020 | 0.010 | 0.002 |
| Nuclease (SEQ ID NO:5 base on active protein | 0.002 | - | - | 0.001 | 0.002 | 0.002 |
| Tetraacetylehtylenediamine | 3.6 | 4.0 | 3.6 | 4.0 | 2.2 | 1.4 |
| Sodium percabonate | 13.0 | 13.2 | 13.0 | 13.2 | 16.0 | 14.0 |
| Chelant 3 | - | 0.2 | - | 0.2 | - | 0.2 |
| Chelant 2 | 0.2 | - | 0.2 | - | 0.2 | 0.2 |
| $MgSO_4$ | - | 0.42 | - | 0.42 | - | 0.4 |
| Perfume | 0.5 | 0.6 | 0.5 | 0.6 | 0.6 | 0.6 |
| Suds suppressor agglomerate | 0.05 | 0.10 | 0.05 | 0.10 | 0.06 | 0.05 |
| Soap | 0.45 | 0.45 | 0.45 | 0.45 | - | - |
| Acid Violet 50 | 0.04 | - | 0.05 | - | 0.04 | - |
| Violet DD | - | 0.04 | - | 0.05 | - | 0.04 |
| S-ACMC | 0.01 | 0.01 | - | 0.01 | - | - |

(continued)

| Ingredient | 25 | 26 | 27 | 28 | 29 | 30 |
|---|---|---|---|---|---|---|
| | % weight | | | | | |
| Direct Violet 9 (active) | - | - | 0.0001 | 0.0001 | - | - |
| Acyl hydrazone | 0.7 | 0.1 | 0.6 | 0.3 | 0.06 | 0.3 |
| Sulfate/ Water & Miscellaneous | Balance | | | | | |

Examples 31-37: Heavy Duty Liquid laundry detergent compositions.

| Ingredients | 31 | 32 | 33 | 34 | 35 | 36 | 37 |
|---|---|---|---|---|---|---|---|
| | % weight | | | | | | |
| $AE_{1.8}S$ | 6.77 | 5.16 | 1.36 | 1.30 | - | - | - |
| $AE_3S$ | | | - | - | 0.45 | - | - |
| LAS | 0.86 | 2.06 | 2.72 | 0.68 | 0.95 | 1.56 | 3.55 |
| HSAS | 1.85 | 2.63 | 1.02 | - | - | - | - |
| AE9 | 6.32 | 9.85 | 10.20 | 7.92 | | | |
| AE8 | | | | | | | 35.45 |
| AE7 | | | | | 8.40 | 12.44 | |
| $C_{12-14}$ dimethyl Amine Oxide | 0.30 | 0.73 | 0.23 | 0.37 | - | - | - |
| $C_{12-18}$ Fatty Acid | 0.80 | 1.90 | 0.60 | 0.99 | 1.20 | - | 15.00 |
| Citric Acid | 2.50 | 3.96 | 1.88 | 1.98 | 0.90 | 2.50 | 0.60 |
| Optical Brightener 1 | 1.00 | 0.80 | 0.10 | 0.30 | 0.05 | 0.50 | 0.001 |
| Optical Brightener 3 | 0.001 | 0.05 | 0.01 | 0.20 | 0.50 | - | 1.00 |
| Sodium formate | 1.60 | 0.09 | 1.20 | 0.04 | 1.60 | 1.20 | 0.20 |
| DTI 1 | 0.32 | 0.05 | - | 0.60 | 0.10 | 0.60 | 0.01 |
| DTI 2 | 0.32 | 0.10 | 0.60 | 0.60 | 0.05 | 0.40 | 0.20 |
| Sodium hydroxide | 2.30 | 3.80 | 1.70 | 1.90 | 1.70 | 2.50 | 2.30 |
| Monoethanolamine | 1.40 | 1.49 | 1.00 | 0.70 | - | - | - |
| Diethylene glycol | 5.50 | - | 4.10 | - | - | - | - |
| Chelant 1 | 0.15 | 0.15 | 0.11 | 0.07 | 0.50 | 0.11 | 0.80 |
| 4-formyl-phenylboronic acid | - | - | - | - | 0.05 | 0.02 | 0.01 |
| Sodium tetraborate | 1.43 | 1.50 | 1.10 | 0.75 | - | 1.07 | - |
| Ethanol | 1.54 | 1.77 | 1.15 | 0.89 | - | 3.00 | 7.00 |
| Polymer 1 | 0.10 | - | - | - | - | - | 2.00 |
| Polymer 2 | 0.30 | 0.33 | 0.23 | 0.17 | - | - | - |
| Polymer 3 | - | - | - | - | - | - | 0.80 |
| Polymer 4 | 0.80 | 0.81 | 0.60 | 0.40 | 1.00 | 1.00 | - |
| 1,2-Propanediol | - | 6.60 | - | 3.30 | 0.50 | 2.00 | 8.00 |
| Structurant | 0.10 | - | - | - | - | - | 0.10 |

(continued)

| Ingredients | 31 | 32 | 33 | 34 | 35 | 36 | 37 |
|---|---|---|---|---|---|---|---|
| | % weight | | | | | | |
| Perfume | 1.60 | 1.10 | 1.00 | 0.80 | 0.90 | 1.50 | 1.60 |
| Perfume encapsulate | 0.10 | 0.05 | 0.01 | 0.02 | 0.10 | 0.05 | 0.10 |
| Protease | 0.80 | 0.60 | 0.70 | 0.90 | 0.70 | 0.60 | 1.50 |
| Galactanase of any of SEQ ID Nos: 1-3 | 0.07 | 0.05 | 0.045 | 0.06 | 0.04 | 0.045 | 0.10 |
| Amylase 1 | 0.30 | - | 0.30 | 0.10 | - | 0.40 | 0.10 |
| Amylase 2 | - | 0.20 | 0.10 | 0.15 | 0.07 | - | 0.10 |
| Xyloglucanase | 0.20 | 0.10 | - | - | 0.05 | 0.05 | 0.20 |
| Lipase | 0.40 | 0.20 | 0.30 | 0.10 | 0.20 | - | - |
| Polishing enzyme | - | 0.04 | - | - | - | 0.004 | - |
| Nuclease | 0.05 | 0.03 | 0.01 | 0.03 | 0.03 | 0.003 | 0.003 |
| Dispersin B | - | - | - | 0.05 | 0.03 | 0.001 | 0.001 |
| Acid Violet 50 | 0.05 | - | - | - | - | - | 0.005 |
| Direct Violet 9 | - | - | - | - | - | 0.05 | - |
| Violet DD | - | 0.035 | 0.02 | 0.037 | 0.04 | - | - |
| Water insoluble plant fiber | 0.2 | - | - | - | 1.2 | - | - |
| Dye control agent | - | 0.3 | - | 0.5 | - | 0.3 | - |
| Alkoxylated polyaryl/ polyalkyl phenol | - | - | 1.2 | - | - | - | 3.1 |
| Water, dyes & minors | Balance | | | | | | |
| pH | 8.2 | | | | | | |

Based on total cleaning and/or treatment composition weight. Unless indicated otherwise, enzyme levels are reported as raw material.

| | |
|---|---|
| Acyl hydrazone | Acyl hydrazone in accordance with the invention, for example 4-(2-(2-((2-hydroxyphenylmethyl)methylene)-hydrazinyl)-2-oxoethyl)-4-methylchloride suppled as Tinocat® LT (BASF) |
| AE1.8S | is $C_{12-15}$ alkyl ethoxy sulfate with an average degree of ethoxylation of 1.8 |
| AE3S | is $C_{12-15}$ alkyl ethoxy sulfate with an av degree of ethoxylation of 3 |
| AE7 | is $C_{12-13}$ alcohol ethoxylate, with an average degree of ethoxylation of 7 |
| AE8 | is $C_{12-13}$ alcohol ethoxylate, with an average degree of ethoxylation of 8 |
| AE9 | is $C_{12-13}$ alcohol ethoxylate, with an average degree of ethoxylation of 9 |
| Alkoxylated polyaryl / polyalkyl phenol | is alkoxylated polyaryl/polyalkyl phenol for example Emulsogen® TS160, Hostapal® BV conc., Sapogenat® T110 or Sapogenat® T139, all from Clariant |
| Amylase 1 | is Stainzyme®, 15 mg active/g |
| Amylase 2 | is Natalase®, 29 mg active/g |
| Amylase 3 | is Stainzyme® Plus, 20 mg active/g, |
| AS | is $C_{12-14}$ alkylsulfate |
| Cellulase 2 | is Celluclean™, 15.6 mg active/g |
| Xyloglucanase | is Whitezyme®, 20mg active/g |
| Chelant 1 | is diethylene triamine pentaacetic acid |
| Chelant 2 | is 1-hydroxyethane 1,1-diphosphonic acid |
| Chelant 3 | is sodium salt of ethylenediamine-N,N'-disuccinic acid, (S,S) isomer (EDDS) |
| Dispersin B | is a glycoside hydrolase, reported as 1000mg active/g |
| DTI 1 | is poly(4-vinylpyridine-1-oxide) (such as Chromabond S-403E®), |
| DTI 2 | is poly(1-vinylpyrrolidone-co-1-vinylimidazole) (such as Sokalan HP56®). |

| | |
|---|---|
| Dye Control Agent | is for example Suparex® O.IN (M1), Nylofixan® P (M2), Nylofixan® PM (M3), or Nylofixan® HF (M4) |
| HSAS | is mid-branched alkyl sulfate as disclosed in US 6,020,303 and US6,060,443 |
| LAS | is linear alkylbenzenesulfonate having an average aliphatic carbon chain length $C_9$-$C_{15}$ (HLAS is acid form). |
| Galactanase | is SEQ ID NO: 3, as active protein. |
| Lipase | is Lipex®, 18 mg active/g |
| Mannanase | is SEQ ID NO: 10, as active protein. |
| Optical Brightener 1 | is disodium 4,4'-bis{[4-anilino-6-morpholino-s-triazin-2-yl]-amino}-2,2'-stilbenedisulfonate |
| Optical Brightener 2 | is disodium 4,4'-bis-(2-sulfostyryl)biphenyl (sodium salt) |
| Optical Brightener 3 | is Optiblanc SPL10® from 3V Sigma |
| Perfume encapsulate | is a core-shell melamine formaldehyde perfume microcapsules |
| Photobleach | is a sulfonated zinc phthalocyanine |
| Polishing enzyme | is Para-nitrobenzyl esterase, reported as 1000mg active/g |
| Polyetheramine | as described in present disclosure. |
| Polymer 1 | is bis($(C_2H_5O)(C_2H_4O)n$)($CH_3$)-$N^+$-$C_xH_{2x}$-$N^+$-($CH_3$)-bis($(C_2H_5O)(C_2H_4O)n$), wherein n = 20-30,x = 3 to 8 or sulphated or sulfonated variants thereof |
| Polymer 2 | is ethoxylated ($EO_{15}$) tetraethylene pentamine |
| Polymer 3 | is ethoxylated polyethylenimine |
| Polymer 4 | is ethoxylated hexamethylene diamine |
| Polymer 5 | is Acusol 305, provided by Rohm&Haas |
| Polymer 6 | is a polyethylene glycol polymer grafted with vinyl acetate side chains, provided by BASF. |
| Protease | is Purafect Prime®, 40.6 mg active/g |
| Protease 2 | is Savinase®, 32.89 mg active/g |
| Protease 3 | is Purafect®, 84 mg active/g |
| Quaternary ammonium | is $C_{12-14}$ Dimethylhydroxyethyl ammonium chloride |
| S-ACMC | is Reactive Blue 19 Azo-CM-Cellulose provided by Megazyme |
| Soil release agent | is Repel-o-tex® SF2, supplied by Solvay |
| Structurant | is Hydrogenated Castor Oil |
| Violet DD | is a thiophene azo polymeric hueing dye provided by Milliken |

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

## Claims

**1.** A cleaning composition comprising:

an endo-beta-1,6-galactanase enzyme;
a peroxygen source; and
an acyl hydrazone bleach catalyst.

**2.** A cleaning composition according to claim 1, wherein the enzyme has an amino acid sequence having at least 60%, or at least 80%, or at least 90% or at least 95% identity with the amino acid sequence shown in SEQ ID NO:1, SEQ ID NO:2 or SEQ ID NO:3.

**3.** A cleaning composition according to claims 1 and 2, wherein the galactanase enzyme is selected from Glycoside Hydrolase Family 30.

**4.** A cleaning composition according to any preceding claim, wherein the galactanase enzyme is obtainable from *Streptomyces davawensis, Trichoderma harzianum, Streptomyces avermitilis,* or a mixture thereof.

**5.** A cleaning composition according to any preceding claim, wherein the composition further comprises a β-N-acetylglucosaminidase enzyme from E.C. 3.2.1.52, preferably an enzyme having at least 70% identity to SEQ ID NO:9.

**6.** A cleaning composition according to any preceding claim, wherein the peroxygen source is $H_2O_2$ or a substance that releases $H_2O_2$ in water.

**7.** A cleaning composition according to any preceding claim, wherein the peroxygen source is selected from the group consisting of perborates, percarbonates, perhydrates, peroxycarboxylic acids, persulfates, preoxydisulfates, diacyl peroxides, tetraacyl diperoxides, and combinations thereof, preferably wherein the peroxygen source is selected from the group consisting of alkali metal perborates, alkali metal percarbonates, urea perhydrates, and combination thereof.

**8.** A cleaning composition according to claim 7, wherein the peroxygen source comprises a peroxycarboxylic acid selected from diperoxydecanedicarboxylic acid, phthalimido peroxycaproic acid, or combinations thereof.

**9.** A cleaning composition according to any preceding claim, wherein the acyl hydrazone bleach catalyst has a structure according to Formula I:

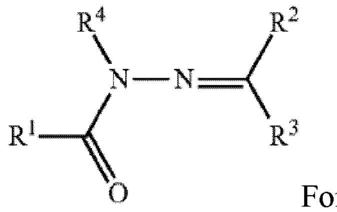

Formula I,

wherein $R^1$ stands for a $CF_3$ or for a $C_{1-28}$ alkyl, $C_{2-28}$ alkenyl, $C_{2-22}$ alkynyl, $C_{3-12}$ cycloalkyl, $C_{3-12}$ cycloalkenyl, phenyl, naphthyl, $C_{7-9}$ aralkyl, $C_{3-20}$ heteroalkyl or $C_{3-12}$ cycloheteroalkyl group,
$R^2$ and $R^3$ independently of one another stand for hydrogen or an optionally substituted $C_{1-28}$ alkyl, $C_{2-28}$ alkenyl, $C_{2-22}$ alkynyl, $C_{3-12}$ cycloalkyl, $C_{3-12}$ cycloalkenyl, $C_{7-9}$ aralkyl, $C_{3-28}$ heteroalkyl, $C_{3-12}$ cycloheteroalkyl, $C_{5-16}$ heteroaralkyl, phenyl, naphthyl or heteroaryl group or $R^2$ and $R^3$ together with the carbon atom linking them stand for an optionally substituted 5-, 6-, 7-, 8- or 9-membered ring that can optionally comprise heteroatoms, and $R^4$ stands for hydrogen or a $C_{1-28}$ alkyl, $C_{2-28}$ alkenyl, $C_{2-22}$ alkynyl, $C_{3-12}$ cycloalkyl, $C_{3-12}$ cycloalkenyl, $C_{7-9}$ aralkyl, $C_{3-20}$ heteroalkyl, $C_{3-12}$ cycloheteroalkyl, $C_{5-16}$ heteroaralkyl group or an optionally substituted phenyl or naphthyl or heteroaryl group.

A cleaning composition according to any preceding claim, wherein $R^1$ stands for a $C_{3-12}$ cycloheteroalkyl group, $R^2$ and $R^3$ independently of one another stand for hydrogen or an optionally substituted phenyl group, and $R^4$ stands for hydrogen.
A liquid cleaning composition according to any preceding claim, wherein the acyl hydrazone bleach catalyst comprises 4-(2-(2-((2-hydroxyphenylmethyl)methylene)-hydrazinyl)-2-oxoethyl)-4-methylchloride according to Formula II:

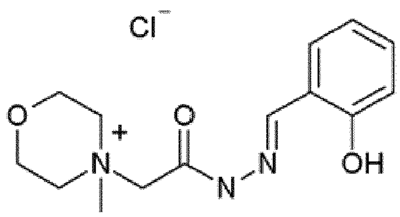

Formula II.

A cleaning composition according to any preceding claim, wherein the cleaning composition further comprises a cleaning cellulase, preferably wherein the cleaning cellulase is an endoglucanase.
A cleaning composition according to any preceding claim, wherein the composition further comprises encapsulates, wherein the encapsulates comprises a shell surrounding a core, the core comprising a benefit agent, preferably the benefit agent comprising perfume.
A method of cleaning a surface, preferably a textile, comprising mixing the cleaning composition according to any preceding claim with water to form an aqueous liquor and contacting a surface, preferably a textile, with the aqueous liquor in a laundering step. The use of an endo-beta-1,6-galactanase enzyme, a peroxygen source and an acyl hydrazone bleach catalyst in a cleaning composition to enhance the stain-removal, bleaching, and/or malodor-reducing benefits from a surface, preferably a textile surface.

Europäisches Patentamt
European Patent Office
Office européen des brevets

# EUROPEAN SEARCH REPORT

Application Number
EP 17 20 4737

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y,D | WO 2015/185689 A1 (NOVOZYMES AS [DK]) 10 December 2015 (2015-12-10)<br>* page 1 - page 2; claims; examples; sequence 2 *<br>* page 3, paragraph 2 *<br>* page 4, paragraph 2-3 *<br>* page 5, paragraph 4 - page 6, paragraph 1 *<br>* page 10, paragraph 2 - page 11, paragraph 3 *<br>* page 12, paragraph 2-5 *<br>* page 16, lines 16-20 *<br>* page 38, last paragraph - page 39, paragraph 3 *<br>* page 42, paragraph 2 - page 43, line 10 *<br>* page 72 - page 78 *<br>----- | 1-15 | INV.<br>C11D3/386<br>C11D3/39 |
| Y | US 2016/066575 A1 (HATZELT ANDRE [DE] ET AL) 10 March 2016 (2016-03-10)<br>* paragraphs [0002], [0007] - [0011], [0014] - [0018], [0021], [0025], [0030]; claims; examples *<br>----- | 1-15 | |
| | | | TECHNICAL FIELDS SEARCHED (IPC) |
| | | | C11D |
| Y | WO 2016/090472 A1 (UNIV CONCORDIA [CA]) 16 June 2016 (2016-06-16)<br>* paragraphs [0002], [0105] *<br>----- | 5 | |

The present search report has been drawn up for all claims

| Place of search | Date of completion of the search | Examiner |
|---|---|---|
| The Hague | 9 April 2018 | Marttin, Emmeline |

2

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 17 20 4737

This annex lists the patent family members relating to the patent documents cited in the above-mentioned European search report. The members are as contained in the European Patent Office EDP file on
The European Patent Office is in no way liable for these particulars which are merely given for the purpose of information.

09-04-2018

| Patent document cited in search report | Publication date | Patent family member(s) | Publication date |
|---|---|---|---|
| WO 2015185689 A1 | 10-12-2015 | CN 106414698 A | 15-02-2017 |
| | | EP 3152290 A1 | 12-04-2017 |
| | | WO 2015185689 A1 | 10-12-2015 |
| US 2016066575 A1 | 10-03-2016 | DE 102012219359 A1 | 24-04-2014 |
| | | EP 2912156 A1 | 02-09-2015 |
| | | US 2015218494 A1 | 06-08-2015 |
| | | US 2016066575 A1 | 10-03-2016 |
| | | WO 2014063857 A1 | 01-05-2014 |
| WO 2016090472 A1 | 16-06-2016 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2015185689 A
- US 20120135498 A
- WO 2015040159 A
- WO 2004067737 A
- WO 2015091989 A
- WO 2015091990 A
- WO 2015024739 A
- WO 2015143360 A
- US 6312936 B1
- US 5679630 A
- US 4760025 A
- US 7262042 B
- WO 09021867 A
- DE 102006022216 A1
- DE 102006022224 A1
- WO 2015089447 A
- WO 2015089441 A
- WO 2016066756 A
- WO 2016066757 A
- WO 2016069557 A
- WO 2016069563 A
- WO 2016069569 A
- WO 8906270 A
- WO 05052161 A
- WO 05052146 A
- WO 07044993 A2
- WO 2014194032 A
- WO 2014194054 A
- WO 2014194117 A
- WO 2015193488 A
- WO 2016075078 A
- WO 9217577 A
- US 5352604 A
- WO 2009149144 A
- WO 2009149145 A
- WO 201056653 A
- WO 201056640 A
- WO 2011072117 A
- US 20110237487 A
- WO 2011140316 A
- WO 2012151480 A
- EP 2510092 A
- EP 2566960 A
- EP 2705145 A
- US 7153818 B
- WO 9700324 A
- EP 1022334 A
- WO 9402597 A
- WO 9418314 A
- WO 9623874 A
- WO 9743424 A
- US 5856164 A
- WO 9923211 A
- WO 9623873 A
- WO 0060060 A
- WO 06002643 A
- US 6093562 A
- WO 09149130 A
- EP 2540825 A
- EP 2357220 A
- EP 2534233 A
- WO 2009100102 A
- WO 2010115028 A
- US 6939702 B1
- US 20090217464 A
- EP 12001034 A
- EP 2623586 A
- US 7141403 B2
- WO 2002099091 A
- WO 01062903 A
- WO 9902663 A
- WO 01064853 A
- WO 2002077242 A
- WO 03089598 A
- WO 9905243 A
- WO 9905242 A
- WO 9905244 A
- WO 9905082 A
- WO 9905084 A
- WO 9905241 A
- WO 9907656 A
- WO 0023549 A
- WO 0023548 A
- WO 0887497 A
- WO 9108281 A
- WO 9001815 A
- US 6020303 A
- US 6060443 A

### Non-patent literature cited in the description

- **A. B. BORASTON et al.** *Biochemical Journal,* 2004, vol. 382, 769-781

- *Biochem J.,* 1991, vol. 280, 309-316
- **NEEDLEMAN ; WUNSCH.** *J. Mol. Biol.,* 1970, vol. 48, 443-453
- **RICE et al.** EMBOSS: The European Molecular Biology Open Software Suite. *Trends in Genetics,* 2000, vol. 16, 276-277